# EUROPEAN PATENT APPLICATION

(11) **EP 4 272 729 A1**
(43) Date of publication of application: **08.11.2023**
(21) Application number: 20967573.5
(22) Date of filing: 30.12.2020
(51) Int. Cl.: A61K 9/08, A61K 38/47, C07K 16/06, A61K 39/395

(54) **RECOMBINANT HUMAN HYALURONIDASE FORMULATION AND APPLICATION THEREOF**

(71) Applicant: Shanghai Bao Pharmaceuticals Co., Ltd., Shanghai 201908 (CN)
(72) Inventor: WANG, Zheng, Shanghai 201908 (CN); LIU, Yanjun, Shanghai 201908 (CN)
(74) Representative: Zwicker, Jörk
(86) International application number: PCT/CN2020/141546
(87) International publication number: WO 2022/141232

(57) **Abstract**

Provided are a recombinant human hyaluronidase formulation and an application thereof, the recombinant human hyaluronidase formulation comprising: recombinant human hyaluronidase, a buffer, a stabilizer, and a non-ionic surfactant, the stabilizer being selected from one or more of trehalose, sucrose, mannitol, sodium chloride, and methionine, and having the ability to maintain the stability of recombinant human hyaluronidase under 2-8 °C conditions; the hyaluronidase formulation can be applied in the preparation of a drug assisting subcutaneous infusion; same can enable a large volume infusion drug originally only able to be administered intravenously to be administered by means of subcutaneous infusion, and is more convenient, safe, and comfortable in clinical use.

## Description

### Technical Field

The present invention relates to the technical field of biomedicine, and in particular to a recombinant human hyaluronidase (HAase) formulation and use thereof in the formulation of a subcutaneous injection promoting agent.

### Background

Hyaluronic acid (HA), also known as hyaluronan, is a linear high-molecular-weight glycosaminoglycan composed of repeatedly connected disaccharide units D-glucuronic acid and N-glucosamine units. It is widely found in connective tissues, mucous tissues, lens of eye and skin in vertebrates, and particularly abundant in embryos, cartilage, synovial fluid, vitreous body, umbilical cord, rooster comb, and other tissues. HA is the most widely distributed acidic mucopolysaccharide in human tissue matrices, and has many functions in the body. For example, HA can form a variety of matrices, restrict the diffusion of water and other extracellular substances, adjust the osmotic pressure, regulate the transport of macromolecular substances, and form a physical barrier around cells, etc. HA is filled between the collagen fiber structures in the extracellular matrix by forming a network barrier in the body, and thus limits the subcutaneous diffusion of drugs, reduces the speed of drugs reaching blood vessels, and limits the volume of liquid injected subcutaneously.

Hyaluronidase (HAase) is an endoproteinase that can specifically hydrolyze HA to improve the permeability of a liquid in tissues. It is widely distributed in serum, brain, placenta and other tissues and body fluids in the body. HA is hydrolyzed by HAase by breaking a glycosidic bond between C1 of glucosamine and C4 of glucuronic acid. This temporarily reduces the viscosity of intercellular matrix, promotes the subcutaneous injection, and accelerates the diffusion of exudate or blood accumulated locally, thus facilitating the absorption.

The commercially available HAase is often obtained by salt precipitation of the testicle of mammals and then lyophilization, or by salt precipitation, removal of pyrogen and then lyophilization. For example, the following materials can be used as a raw material in the purification process: testicular material of mammals after one salt precipitation operation and then lyophilization, testicular material after two salt precipitation operations and then lyophilization, or testicular material after two salt precipitation operations, removal of pyrogen and then lyophilization. The existing HAase is extracted from sheep testicle by two salt precipitation operations, lyophilization, dialysis, removal of pyrogen, and then lyophilization, and the extracted material is mixed with a large amount of proteins other than HAase. As a crude product, the commercially available lyophilized HAase contains numerous impurities, and has low purity and thus poor stability when it is prepared into a liquid formulation.

Intravenous infusion refers to a method of delivering a large volume of a sterile liquid, electrolyte and drug into the body via the vein. Although the drug can be administered to the patients quickly and directly to achieve a therapeutic concentration, intravenous infusion has obvious adverse reactions. The adverse reactions caused by intravenous infusion mainly include adverse drug reactions, fainting during acupuncture, phlebitis, heavy circulating load, air embolism, infection, and others, causing pain to the patients and hindrance to the treatment. Serious adverse reactions resulting from infusion may even be life-threatening. According to incomplete statistics, the annual infusion volume exceeds 10 billion bottles, and about 200,000 deaths can be attributed to adverse reactions to infusion every year in China. The global average infusion volume per person is 2.5-3.3 bottles/year, while it is 8 bottles in China. Since 2015, in order to reduce the occurrence of medical accidents caused by intravenous infusion, regulations on restricting and regulating the treatment by intravenous infusion have successively issued in many provinces.

Therefore, to facilitate the clinical use and reduce the risks, there is an urgent need for a safe, effective and comfortable injection method to replace the traditional intravenous infusion.

### Summary of the Invention

To overcome the defects existing in the prior art, the present invention provides a recombinant human hyaluronidase formulation and use thereof in the formulation of subcutaneous infusion promoting agent. The formulation has the advantages of convenience during clinical use, wide use, simple ingredients, good stability, low irritation, safety and effectiveness of the product. By using the recombinant human HAase formulation, the problem existing in clinical treatment that intravenous infusion is necessitated due to the difficulty in large volume injection by subcutaneous administration is solved. In the present invention, it is unexpectedly found, in an animal test where the subcutaneous injection of a drug is promoted by recombinant human HAase, that the drug solution, used in combination with HAase, can be rapidly diffused and absorbed after being subcutaneously injected into miniature pigs. Compared with the negative control group (directly subcutaneously injected with the drug solution without combining with HAase), in the test group, the subcutaneous injection pressure of the drug solution is significantly reduced, the degree of swelling and deformation of the skin after injection and the time required to return to normal are reduced, the injection site has no obvious redness and swelling, and the drug solution promoted for injection is rapidly diffused and absorbed, making it possible to subcutaneously administer a large volume of drug solution.

The following technical solutions are adopted in the present invention to solve the above technical problems.

### 1. HAase formulation and use

In a first aspect, the present invention relates to a recombinant human HAase formulation, which includes recombinant human HAase, a buffering agent, a stabilizer and a nonionic surfactant, where
the recombinant human HAase has an enzyme activity of 45 units/ml to 4500000 units/ml;
the buffering agent has a concentration of 5 to 50 mM;
the stabilizer has a concentration of 1 to 500 mM, and the stabilizer is one or more selected from trehalose, sucrose, mannitol, sodium chloride, and methionine;
the nonionic surfactant has a concentration of 0.01% to 0.1% (w/v); and
the formulation has a pH of 5.5 to 8.0.

The enzyme activity of the recombinant human HAase is preferably 45 units/ml to 3000000 units/ml; further preferably 45 units/ml to 1500000 units/ml; and still further preferably 45 units/ml to 300000 units/ml, for example, 45 units/ml, 100 units/ml, 150 units/ml, 500 units/ml, 1000 units/ml, 5000 units/ml, 50000 units/ml, 300000 units/ml, 1500000 units/ml, 3000000 units/ml, or 4500000 units/ml.

The concentration of the recombinant human HAase is preferably 0.15 to 0.75 µg/ml, 0.75 to 2.5 µg/ml, 2.5 to 5 µg/ml, 5.5 to 25 µg/ml, 25 µg/ml to 0.3 mg/ml, 0.3 to 1.5 mg/ml, 1.5 to 7.5 mg/ml, 7.5 to 15 mg/ml, or 15 to 22.5 mg/ml, for example, 0.15 µg/ml, 0.33 µg/ml, 0.5 µg/ml, 1.7 µg/ml, 3.3 µg/ml, 17 µg/ml, 0.2 mg/ml, 1 mg/ml, 5 mg/ml, 10 mg/ml, or 15 mg/ml.

The enzyme activity of the recombinant human HAase may further be 300000 units/ml to 1500000 units/ml, 1500000 units/ml to 3000000 units/ml, or 300000 units/ml to 450000 units/ml.

Preferably, the pH achieved by the buffering agent is 5.5 to 8.0, for example, 5.5, 6.0, 6.5, 7.0, 7.5 or 8.0.

The buffering agent can be conventional in the art, and is preferably one or more of a histidine buffering agent, an acetic acid buffering agent, a phosphate buffering agent, a citric acid buffering agent, and a Tris buffering agent, for example, a histidine buffering agent, an acetic acid buffering agent, a phosphate buffering agent, a citric acid buffering agent or a Tris buffering agent.

The pH achieved by the histidine buffering agent is 5.5 to 7.5, the pH achieved by the acetic acid buffering agent is 5.0 to 6.0, the pH achieved by the phosphate buffering agent is 6.0 to 8.0, the pH achieved by the citric acid buffering agent is 5.0 to 7.0, and the pH achieved by the Tris buffering agent is 7.0 to 8.0.

Preferably, the histidine buffering agent has a pH of 6.0.

Preferably, the acetic acid buffering agent has a pH of 5.0.

Preferably, the phosphate buffering agent has a pH of 7.0.

Preferably, the citric acid buffering agent has a pH of 6.5.

Preferably, the Tris buffering agent has a pH of 8.0.

Preferably, the histidine buffering agent has a concentration of 5 to 50 mM, for example, 5 mM, 10 mM or 50 mM.

Preferably, the acetic acid buffering agent has a concentration of 5 to 50 mM, for example, 5 mM, 10 mM or 50 mM.

Preferably, the phosphate buffering agent has a concentration of 5 to 50 mM, for example, 5 mM, 10 mM or 50 mM.

Preferably, the citric acid buffering agent has a concentration of 5 to 50 mM, for example, 5 mM, 10 mM or 50 mM.

Preferably, the Tris buffering agent has a concentration of 5 to 50 mM, for example, 5 mM, 10 mM or 50 mM.

Preferably, the stabilizer is one or more selected from mannitol, sucrose, trehalose, sodium chloride and methionine. For example, the stabilizer comprises methionine, and two or more selected from the group consisting of trehalose, sucrose, mannitol and sodium chloride.

The concentration of the stabilizer is preferably 100 to 400 mM, and further preferably 150 to 350 mM, for example, , 155 mM, 188 mM, 193 mM, 215 mM, 233 mM, 240 mM, 263 mM, 283 mM, 292 mM, 316 mM or 330 mM.

Preferably, the trehalose has a concentration of 25 to 200 mM, for example, 25 mM, 53 mM, 132 mM or 200 mM.

Preferably, the sucrose has a concentration of 20 to 200 mM, for example, 25 mM, 53 mM, 132 mM or 200 mM. Preferably, the sodium chloride has a concentration of 30 to 250 mM, for example, 30 mM, 50 mM, 90 mM, 130 mM, 145 mM, 170 mM or 250 mM.

Preferably, the methionine has a concentration of 5 to 50 mM, for example, 5 mM, 10 mM or 50 mM.

Preferably, the mannitol has a concentration of 150 to 300 mM, for example, 165 mM, 220 mM or 280 mM.

The recombinant human HAase formulation of the present invention can be used as a liquid formulation or lyophilized into a lyophilized formulation. In a preferred embodiment of the present invention, the stabilizer has a concentration of 185 to 380 mM, and includes at least mannitol and methionine, as well as trehalose or sucrose, where the mannitol has a concentration of 150 to 280 mM, the methionine has a concentration of 5 to 50 mM, and the trehalose or sucrose has a concentration of 25 to 130 mM. The formulation according to this embodiment is more suitably prepared into a lyophilized formulation.

The nonionic surfactant is one or more selected from polysorbate 20, polysorbate 80 and Poloxamer188.

Preferably, the nonionic surfactant has a concentration of 0.02% (w/v).

In a preferred embodiment of the present invention, the recombinant human HAase formulation has a composition selected from:
In a specific embodiment of the present invention, the stabilizer useful in the recombinant human HAase formulation has a composition selected from:
(1) 145 mM sodium chloride and 10 mM methionine;
(2) 130 mM sodium chloride, 53 mM trehalose and 10 mM methionine;
(3) 130 mM sodium chloride, 53 mM sucrose and 10 mM methionine;
(4) 26 mM trehalose, 280 mM mannitol and 10 mM methionine.
(5) 30 mM sodium chloride, 200 mM trehalose and 10 mM methionine;
(6) 180 mM sodium chloride, 25 mM trehalose and 10 mM methionine;
(7) 30 mM sodium chloride, 200 mM sucrose and 10 mM methionine;
(8) 180 mM sodium chloride, 25 mM sucrose and 10 mM methionine;
(9) 130 mM sodium chloride, 53 mM trehalose and 5 mM methionine;
(10) 130 mM sodium chloride, 53 mM trehalose and 50 mM methionine;
(11) 130 mM sodium chloride, 53 mM trehalose and 10 mM methionine;
(12) 22 mM mannitol, 53 mM trehalose and 10 mM methionine;
(13) 53 mM trehalose, 220 mM mannitol and 10 mM methionine;
(14) 53 mM trehalose, 220 mM mannitol and 5 mM methionine;
(15) 53 mM trehalose, 220 mM mannitol and 50 mM methionine;
(16) 150 mM mannitol, 170 mM sodium chloride and 10 mM methionine;
(17) 220 mM mannitol, 53 mM trehalose and 10 mM methionine;
(18) 150 mM mannitol, 50 mM sodium chloride, 53 mM trehalose and 10 mM methionine;
(19) 150 mM mannitol, 90 mM sodium chloride, 130 mM trehalose and 10 mM methionine;
(20) 150 mM mannitol, 25 mM trehalose and 10 mM methionine;
(21) 220 mM mannitol, 53 mM sucrose and 10 mM methionine;
(22) 150 mM mannitol, 50 mM sodium chloride, 53 mM sucrose and 10 mM methionine;
(23) 150 mM mannitol, 90 mM sodium chloride, 130 mM sucrose and 10mM methionine;
(24) 150 mM mannitol, 25 mM sucrose and 10 mM methionine; and
(25) 132 mM trehalose, 150 mM mannitol and 10 mM methionine.

The recombinant human HAase is human testicular HAase, and preferably an extramembranous domain of human testicular HAase.

The recombinant human HAase includes an amino acid sequence as shown in SEQ ID NO: 1, for example, PH20, having an amino acid sequence as shown in SEQ ID NO: 2.
SEQ ID NO: 1 
LNFRAPPVIPNVPFLWAWNAPSEFCLGKFDEPLDMSLFSFIGSPRINATGQGVTIFY
VDRLGYYPYIDSITGVTVNGGIPQKISLQDHLDKAKKDITFYMPVDNLGMAVIDWEEW
RPTWARNWKPKDVYKNRSIELVQQQNVQLSLTEATEKAKQEFEKAGKDFLVETIKLGK
LLRPNHLWGYYLFPDCYNHHYKKPGYNGSCFNVEIKRNDDLSWLWNESTALYPSIYL
NTQQSPVAATLYVRNRVREAIRVSKIPDAKSPLPVFAYTRIVFTDQVLKFLSQDELVYTF
GETVALGASGIVIWGTLSIMRSMKSCLLLDNYMETILNPYIINVTLAAKMCSQVLCQEQ
GVCIRKNWNSSDYLHLNPDNFAIQLEKGGKFTVRGKPTLEDLEQFSEKFYCSCYSTLSC
KEKADVKDTDAVDVCIADGVCIDAFLKPPMETEE
SEQ ID NO: 2

Preferably, in the present invention, the recombinant HAase is obtained by purification from the cell culture supernatant of CHO cells expressing the recombinant HAase.

Preferably, the recombinant HAase has a purity of> 95%, and a specific activity of greater than 70000 units/mg.

In a preferred embodiment of the present invention, the recombinant human HAase formulation has a composition selected from:
(1) 5000 units/ml recombinant human HAase, 5 mM phosphate buffering agent, 53 mM trehalose, 130 mM sodium chloride, 10 mM methionine and 0.02% (w/v) polysorbate 20, pH 7.0;
(2) 5000 units/ml recombinant human HAase, 10 mM phosphate buffering agent, 53 mM trehalose, 130 mM sodium chloride, 10 mM methionine and 0.02% (w/v) polysorbate 20, pH 7.0;
(3) 5000 units/ml recombinant human HAase, 50 mM phosphate buffering agent, 53 mM trehalose, 130 mM sodium chloride, 10 mM methionine and 0.02% (w/v) polysorbate 20, pH 7.0;
(4) 5000 units/ml recombinant human HAase, 5 mM histidine buffering agent, 53 mM trehalose, 130 mM sodium chloride, 10 mM methionine and 0.02% (w/v) polysorbate 20, pH 6.0;
(5) 5000 units/ml recombinant human HAase, 50 mM histidine buffering agent, 53 mM trehalose, 130 mM sodium chloride, 10 mM methionine and 0.02% (w/v) polysorbate 20, pH 6.0;
(6) 5000 units/ml recombinant human HAase, 5 mM acetic acid buffering agent, 53 mM trehalose, 130 mM sodium chloride, 10 mM methionine and 0.02% (w/v) polysorbate 20, pH 5.0;
(7) 5000 units/ml recombinant human HAase, 50 mM acetic acid buffering agent, 53 mM trehalose, 130 mM sodium chloride, 10 mM methionine and 0.02% (w/v) polysorbate 20, pH 5.0;
(8) 5000 units/ml recombinant human HAase, 5 mM citric acid buffering agent, 53 mM trehalose, 130 mM sodium chloride, 10 mM methionine and 0.02% (w/v) polysorbate 20, pH 6.5;
(9) 5000 units/ml recombinant human HAase, 50 mM citric acid buffering agent, 53 mM trehalose, 130 mM sodium chloride, 10 mM methionine and 0.02% (w/v) polysorbate 20, pH 6.5;
(10) 5000 units/ml recombinant human HAase, 5 mM Tris buffering agent, 53 mM trehalose, 130 mM sodium chloride, 10 mM methionine and 0.02% (w/v) polysorbate 20, pH 8.0;
(11) 5000 units/ml recombinant human HAase, 50 mM Tris buffering agent, 53 mM trehalose, 130 mM sodium chloride, 10 mM methionine and 0.02% (w/v) polysorbate 20, pH 8.0;
(12) 5000 units/ml recombinant human HAase, 10 mM phosphate buffering agent, 53 mM trehalose, 130 mM sodium chloride, 5 mM methionine and 0.02% (w/v) polysorbate 20, pH 7.0;
(13) 5000 units/ml recombinant human HAase, 10 mM phosphate buffering agent, 53 mM trehalose, 130 mM sodium chloride, 50 mM methionine and 0.02% (w/v) polysorbate 20, pH 7.0;
(14) 5000 units/ml recombinant human HAase, 10 mM phosphate buffering agent, 200 mM trehalose, 30 mM sodium chloride, 10 mM methionine and 0.02% (w/v) polysorbate 20, pH 7.0;
(15) 5000 units/ml recombinant human HAase, 10 mM phosphate buffering agent, 53 mM trehalose, 130 mM sodium chloride, 10 mM methionine and 0.02% (w/v) Poloxamer188, pH 7.0;
(16) 5000 units/ml recombinant human HAase, 10 mM phosphate buffering agent, 25 mM trehalose, 180 mM sodium chloride, 10 mM methionine and 0.02% (w/v) polysorbate 20, pH 7.0;
(17) 5000 units/ml recombinant human HAase, 10 mM phosphate buffering agent, 200 mM sucrose, 30 mM sodium chloride, 10 mM methionine and 0.02% (w/v) polysorbate 20, pH 7.0;
(18) 5000 units/ml recombinant human HAase, 10 mM phosphate buffering agent, 53 mM sucrose, 130 mM sodium chloride, 10 mM methionine and 0.02% (w/v) polysorbate 20, pH 7.0;
(19) 5000 units/ml recombinant human HAase, 10 mM phosphate buffering agent, 25 mM sucrose, 180 mM sodium chloride, 10 mM methionine and 0.02% (w/v) polysorbate 20, pH 7.0;
(20) 5000 units/ml recombinant human HAase, 10 mM phosphate buffering agent, 53 mM trehalose, 130 mM sodium chloride, 10 mM methionine and 0.02% (w/v) polysorbate 80, pH 7.0;
(21) 5000 units/ml recombinant human HAase, 10 mM phosphate buffering agent, 53 mM trehalose, 130 mM sodium chloride, 10 mM methionine and 0.01% (w/v) polysorbate 80, pH 7.0;
(22) 5000 units/ml recombinant human HAase, 10 mM phosphate buffering agent, 53 mM trehalose, 130 mM sodium chloride, 10 mM methionine and 0.10% (w/v) polysorbate 80, pH 7.0;
(23) 5000 units/ml recombinant human HAase, 10 mM phosphate buffering agent, 53 mM trehalose, 130 mM sodium chloride, 10 mM methionine and 0.01% (w/v) polysorbate 20, pH 7.0;
(24) 5000 units/ml recombinant human HAase, 10 mM phosphate buffering agent, 53 mM trehalose, 130 mM sodium chloride, 10 mM methionine and 0.10% (w/v) polysorbate 20, pH 7.0;
(25) 5000 units/ml recombinant human HAase, 10 mM phosphate buffering agent, 53 mM trehalose, 130 mM sodium chloride, 10 mM methionine and 0.01% (w/v) Poloxamer188, pH 7.0;
(26) 5000 units/ml recombinant human HAase, 10 mM phosphate buffering agent, 53 mM trehalose, 130 mM sodium chloride, 10 mM methionine and 0.10% (w/v) Poloxamer188, pH 7.0;
(27) 500 units/ml recombinant human HAase, 10 mM phosphate buffering agent, 53 mM trehalose, 130 mM sodium chloride, 10 mM methionine and 0.02% (w/v) polysorbate 20, pH 7.0;
(28) 45 units/ml recombinant human HAase, 10 mM phosphate buffering agent, 53 mM trehalose, 130 mM sodium chloride, 10 mM methionine and 0.02 % (w/v) polysorbate 20, pH 7.0;
(29) 150 units/ml recombinant human HAase, 10 mM phosphate buffering agent, 53 mM trehalose, 130 mM sodium chloride, 10 mM methionine and 0.02% (w/v) polysorbate 20, pH 7.0;
(30) 1000 units/ml recombinant human HAase, 10 mM phosphate buffering agent, 53 mM trehalose, 130 mM sodium chloride, 10 mM methionine and 0.02% (w/v) polysorbate 20, pH 7.0;
(31) 50000 units/ml recombinant human HAase, 10 mM phosphate buffering agent, 53 mM trehalose, 130 mM sodium chloride, 10 mM methionine and 0.02% (w/v) polysorbate 20, pH 7.0;
(32) 300000 units/ml recombinant human HAase, 10 mM phosphate buffering agent, 53 mM trehalose, 130 mM sodium chloride, 10 mM methionine and 0.02% (w/v) polysorbate 20, pH 7.0;
(33) 1500000 units/ml recombinant human HAase, 10 mM phosphate buffering agent, 53 mM trehalose, 130 mM sodium chloride, 10 mM methionine and 0.02% (w/v) polysorbate 20, pH 7.0;
(34) 3000000 units/ml recombinant human HAase, 10 mM phosphate buffering agent, 53 mM trehalose, 130 mM sodium chloride, 10 mM methionine and 0.02% (w/v) polysorbate 20, pH 7.0;
(35) 4500000 units/ml recombinant human HAase, 10 mM phosphate buffering agent, 53 mM trehalose, 130 mM sodium chloride, 10 mM methionine and 0.02% (w/v) polysorbate 20, pH 7.0;
(36) 4500000 units/ml recombinant human HAase, 10 mM phosphate buffering agent, 145 mM sodium chloride, 10 mM methionine and 0.02% (w/v) polysorbate 20, pH 7.0;
(37) 1500000 units/ml recombinant human HAase, 10 mM phosphate buffering agent, 145 mM sodium chloride, 10 mM methionine and 0.02% (w/v) polysorbate 20, pH 7.0;
(38) 300000 units/ml recombinant human HAase, 10 mM phosphate buffering agent, 145 mM sodium chloride, 10 mM methionine and 0.02% (w/v) polysorbate 20, pH 7.0;
(39) 5000 units/ml recombinant human HAase, 10 mM phosphate buffering agent, 145 mM sodium chloride, 10 mM methionine and 0.02% (w/v) polysorbate 20, pH 7.0;
(40) 150 units/ml recombinant human HAase, 10 mM phosphate buffering agent, 145 mM sodium chloride, 10 mM methionine and 0.02% (w/v) polysorbate 20, pH 7.0;
(41) 45 units/ml recombinant human HAase, 10 mM phosphate buffering agent, 145 mM sodium chloride, 10 mM methionine and 0.02% (w/v) polysorbate 20, pH 7.0;
(42) 45 units/ml recombinant human HAase, 10 mM phosphate buffering agent, 22 mM mannitol, 53 mM trehalose, 10 mM methionine and 0.02% polysorbate 20, pH 7.0;
(43) 500 units/ml recombinant human HAase, 10 mM phosphate buffering agent, 22 mM mannitol, 53 mM trehalose, 10 mM methionine and 0.02% polysorbate 20, pH 7.0;
(44) 5000 units/ml recombinant human HAase, 10 mM phosphate buffering agent, 22 mM mannitol, 53 mM trehalose, 10 mM methionine and 0.02% polysorbate 20, pH 7.0;
(45) 50000 units/ml recombinant human HAase, 10 mM phosphate buffering agent, 22 mM mannitol, 53 mM trehalose, 10 mM methionine and 0.02% polysorbate 20, pH 7.0;
(46) 300000 units/ml recombinant human HAase, 10 mM phosphate buffering agent, 22 mM mannitol, 53 mM trehalose, 10 mM methionine and 0.02% polysorbate 20, pH 7.0;
(47) 4500000 units/ml recombinant human HAase, 10 mM phosphate buffering agent, 22 mM mannitol, 53 mM trehalose, 10 mM methionine and 0.02% polysorbate 20, pH 7.0;
(48) 5000 units/ml recombinant human HAase, 5 mM phosphate buffering agent, 53 mM trehalose, 220 mM mannitol, 10 mM methionine and 0.02% polysorbate 20, pH 7.0;
(49) 5000 units/ml recombinant human HAase, 10 mM phosphate buffering agent, 53 mM trehalose, 220 mM mannitol, 10 mM methionine and 0.02% polysorbate 20, pH 7.0;
(50) 5000 units/ml recombinant human HAase, 50 mM phosphate buffering agent, 53 mM trehalose, 220 mM mannitol, 10 mM methionine and 0.02% polysorbate 20, pH 7.0;
(51) 5000 units/ml recombinant human HAase, 5 mM phosphate buffering agent, 53 mM trehalose, 220 mM mannitol, 10 mM methionine and 0.02% polysorbate 20, pH 6.0;
(52) 5000 units/ml recombinant human HAase, 50 mM phosphate buffering agent, 53 mM trehalose, 220 mM mannitol, 10 mM methionine and 0.02% polysorbate 20, pH 6.0;
(53) 5000 units/ml recombinant human HAase, 50 mM acetic acid buffering agent, 53 mM trehalose, 220 mM mannitol, 10 mM methionine and 0.02% polysorbate 20, pH 5.0;
(54) 5000 units/ml recombinant human HAase, 5 mM acetic acid buffering agent, 53 mM trehalose, 220 mM mannitol, 10 mM methionine and 0.02% polysorbate 20, pH 5.0;
(55) 5000 units/ml recombinant human HAase, 50 mM citric acid buffering agent, 53 mM trehalose, 220 mM mannitol, 10 mM methionine and 0.02% polysorbate 20, pH 6.5;
(56) 5000 units/ml recombinant human HAase, 5 mM citric acid buffering agent, 53 mM trehalose, 220 mM mannitol, 10 mM methionine and 0.02% polysorbate 20, pH 6.5;
(57) 5000 units/ml recombinant human HAase, 50 mM Tris buffering agent, 53 mM trehalose, 220 mM mannitol, 10 mM methionine and 0.02% polysorbate 20, pH 8.0;
(58) 5000 units/ml recombinant human HAase, 5 mM Tris buffering agent, 53 mM trehalose, 220 mM mannitol, 10 mM methionine and 0.02% polysorbate 20, pH 8.0;
(59) 5000 units/ml recombinant human HAase, 10 mM phosphate buffering agent, 53 mM trehalose, 220 mM mannitol, 50 mM methionine and 0.02% polysorbate 20, pH 7.0;
(60) 5000 units/ml recombinant human HAase, 10 mM phosphate buffering agent, 53 mM trehalose, 220 mM mannitol, 5 mM methionine and 0.02% polysorbate 20, pH 7.0;
(61) 5000 units/ml recombinant human HAase, 10 mM phosphate buffering agent, 150 mM mannitol, 170 mM sodium chloride, 10 mM methionine and 0.02% polysorbate 20, pH 7.0;
(62) 5000 units/ml recombinant human HAase, 10 mM phosphate buffering agent, 220 mM mannitol, 53 mM trehalose, 10 mM methionine and 0.02% polysorbate 20, pH 7.0;
(63) 5000 units/ml recombinant human HAase, 10 mM phosphate buffering agent, 150 mM mannitol, 50 mM sodium chloride, 53 mM trehalose, 10 mM methionine and 0.02% polysorbate 20, pH 7.0;
(64) 5000 units/ml recombinant human HAase, 10 mM phosphate buffering agent, 150 mM mannitol, 90 mM sodium chloride, 130 mM trehalose, 10 mM methionine and 0.02% polysorbate 20, pH 7.0;
(65) 5000 units/ml recombinant human HAase, 10 mM phosphate buffering agent, 150 mM mannitol, 25 mM trehalose, 10 mM methionine and 0.02% polysorbate 20, pH 7.0;
(66) 5000 units/ml recombinant human HAase, 10 mM phosphate buffering agent, 220 mM mannitol, 53 mM sucrose, 10 mM methionine and 0.02% polysorbate 20, pH 7.0;
(67) 5000 units/ml recombinant human HAase, 10 mM phosphate buffering agent, 150 mM mannitol, 50 mM sodium chloride, 53 mM sucrose, 10 mM methionine and 0.02% polysorbate 20, pH 7.0;
(68) 5000 units/ml recombinant human HAase, 10 mM phosphate buffering agent, 150 mM mannitol, 90 mM sodium chloride, 130 mM sucrose, 10mM methionine and 0.02% polysorbate 20, pH 7.0;
(69) 5000 units/ml recombinant human HAase, 10 mM phosphate buffering agent, 150 mM mannitol, 25 mM sucrose, 10 mM methionine and 0.02% polysorbate 20, pH 7.0;
(70) 5000 units/ml recombinant human HAase, 10 mM phosphate buffering agent, 132 mM trehalose, 150 mM mannitol, 10 mM methionine and 0.02% polysorbate 20, pH 7.0;
(71) 5000 units/ml recombinant human HAase, 10 mM phosphate buffering agent, 26 mM trehalose, 280 mM mannitol, 10 mM methionine and 0.02% polysorbate 20, pH 7.0;
(72) 5000 units/ml recombinant human HAase, 10 mM phosphate buffering agent, 53 mM trehalose, 220 mM mannitol, 10 mM methionine and 0.01% polysorbate 20, pH 7.0;
(73) 5000 units/ml recombinant human HAase, 10 mM phosphate buffering agent, 53 mM trehalose, 220 mM mannitol, 10 mM methionine and 0.10% polysorbate 20, pH 7.0;
(74) 5000 units/ml recombinant human HAase, 10 mM phosphate buffering agent, 53 mM trehalose, 220 mM mannitol, 10 mM methionine and 0.01% polysorbate 80, pH 7.0;
(75) 5000 units/ml recombinant human HAase, 10 mM phosphate buffering agent, 53 mM trehalose, 220 mM mannitol, 10 mM methionine and 0.10% polysorbate 80, pH 7.0;
(76) 5000 units/ml recombinant human HAase, 10 mM phosphate buffering agent, 53 mM trehalose, 220 mM mannitol, 10 mM methionine and 0.02% polysorbate 80, pH 7.0;
(77) 5000 units/ml recombinant human HAase, 10 mM phosphate buffering agent, 53 mM trehalose, 220 mM mannitol, 10 mM methionine and 0.10% Poloxamer 188, pH 7.0;
(78) 5000 units/ml recombinant human HAase, 10 mM phosphate buffering agent, 53 mM trehalose, 220 mM mannitol, 10 mM methionine and 0.01% Poloxamer 188, pH 7.0;
(79) 5000 units/ml recombinant human HAase, 10 mM phosphate buffering agent, 53 mM trehalose, 220 mM mannitol, 10 mM methionine and 0.02% Poloxamer 188, pH 7.0;
(80) 150 units/ml recombinant human HAase, 10 mM phosphate buffering agent, 53 mM trehalose, 220 mM mannitol, 10 mM methionine and 0.02% polysorbate 20, pH 7.0;
(81) 500 units/ml recombinant human HAase, 10 mM phosphate buffering agent, 53 mM trehalose, 220 mM mannitol, 10 mM methionine and 0.02% polysorbate 20, pH 7.0;
(82) 1000 units/ml recombinant human HAase, 10 mM phosphate buffering agent, 53 mM trehalose, 220 mM mannitol, 10 mM methionine and 0.02% polysorbate 20, pH 7.0;
(83) 300000 units/ml recombinant human HAase, 10 mM phosphate buffering agent, 53 mM trehalose, 220 mM mannitol, 10 mM methionine and 0.02% polysorbate 20, pH 7.0;
(84) 50000 units/ml recombinant human HAase, 10 mM phosphate buffering agent, 53 mM trehalose, 220 mM mannitol, 10 mM methionine and 0.02% polysorbate 20, pH 7.0;
(85) 1500000 units/ml recombinant human HAase, 10 mM phosphate buffering agent, 53 mM trehalose, 220 mM mannitol, 10 mM methionine and 0.02% polysorbate 20, pH 7.0;
(86) 3000000 units/ml recombinant human HAase, 10 mM phosphate buffering agent, 53 mM trehalose, 220 mM mannitol, 10 mM methionine and 0.02% polysorbate 20, pH 7.0; and
(87) 4500000 units/ml recombinant human HAase, 10 mM phosphate buffering agent, 53 mM trehalose, 220 mM mannitol, 10 mM methionine and 0.02% polysorbate 20, pH 7.0.

As described above, the recombinant HAase formulation of the present invention can be prepared into a lyophilized form.

Preferably, the cryoprotectant can be any one or a combination of at least two of sucrose, trehalose, maltose or lactose, preferably sucrose and/or trehalose, and most preferably 20 g/L trehalose.

Preferably, the excipient is mannitol and/or glycine, preferably mannitol, and most preferably 40g/L mannitol.

Preferably, the lyophilized formulation is dissolved in water for injection, and then used for subcutaneous injection.

In another aspect, the present invention provides a method for preparing a lyophilized formulation of recombinant HAase according to the first aspect. The method includes the following steps:
A. adding recombinant HAase, a buffering agent, a cryoprotectant, an excipient, an enzyme protectant and a surfactant to water and mixing uniformly to prepare a liquid formulation of recombinant HAase; and
B. lyophilizing the liquid formulation of recombinant HAase obtained in Step A to obtain the lyophilized formulation of recombinant HAase.

Preferably, the lyophilization process includes:
(1) pre-freezing at -2°C to -8°C, for example, -2°C, -3°C, -4°C, -5°C, -6°C, -7°C or -8°C, for 0.5 to 1 hrs, for example, 0.5 hr, 0.6 hr, 0.7 hr, 0.8 hr, 0.9 hr or 1 hr;
(2) pre-freezing at -35°C to -40°C, for example, -35°C, -36°C, -37°C, -38°C, -39°C or - 40°C, for 2 to 4 hrs, for example, 2 hrs, 2.2 hrs, 2.4 hrs, 2.6 hrs, 2.8 hrs, 3 hrs, 3.3 hrs, 3.5 hrs, 3.7 hrs, 3.9 hrs or 4 hrs;
(3) drying at -20°C to -30°C, for example, -20°C, -21°C, -22°C, -23°C, -24°C, -25°C, - 26°C, -27°C, -28°C, -29°C or -30°C, under 10 to 20 mbar, for example, 10 mbar, 11 mbar, 12 mbar, 13 mbar, 14 mbar, 15 mbar, 16 mbar, 17 mbar, 18 mbar, 19 mbar or 20 mbar, for 15 to 25 hrs, for example, 15 hrs, 16 hrs, 17 hrs, 18 hrs, 19 hrs, 20 hrs, 21 hrs, 22 hrs, 23 hrs, 24 hrs or 25 hrs;
(4) drying at -15°C to -25°C, for example, -15°C, -16°C, -17°C, -18°C, -19°C, -20°C, - 21°C, -22°C, -23°C, -24°C or -25°C, under 0.1 to 0.2 mbar, for example, 0.1 mbar, 0.11 mbar, 0.12 mbar, 0.13 mbar, 0.14 mbar, 0.15 mbar, 0.16 mbar, 0.17 mbar, 0.18 mbar, 0.19 mbar or 0.2 mbar, for 0.5 to 3 hrs, for example, 0.5 hr, 0.7 hr, 0.9 hr, 1 hr, 1.3 hrs, 1.5 hrs, 1.8 hrs, 2 hrs, 2.2 hrs, 2.4 hrs, 2.6 hrs, 2.8 hrs or 3 hrs;
(5) drying at -5°C to 10°C, for example, -5°C, -4°C, -3°C, -2°C, -1°C, 0°C, 1°C, 2°C, 3°C, 4°C, 5°C, 6°C, 7°C, 8°C, 9°C or 10°C, under 0.1 to 0.2 mbar, for example, 0.1 mbar, 0.11 mbar, 0.12 mbar, 0.13 mbar, 0.14 mbar, 0.15 mbar, 0.16 mbar, 0.17 mbar, 0.18 mbar, 0.19 mbar or 0.2 mbar, for 0.5 to 3 hrs, for example, 0.5 hr, 0.7 hr, 0.9 hr, 1 hr, 1.3 hrs, 1.5 hrs, 1.8 hrs, 2 hrs, 2.2 hrs, 2.4 hrs, 2.6 hrs, 2.8 hrs or 3 hrs; and
(6) drying at 15°C to 30°C, for example, 15°C, 16°C, 17°C, 18°C, 19°C, 20°C, 22°C, 24°C, 26°C, 28°C, 29°C or 30°C, under 0.1 to 0.2 mbar, for example, 0.1 mbar, 0.11 mbar, 0.12 mbar, 0.13 mbar, 0.14 mbar, 0.15 mbar, 0.16 mbar, 0.17 mbar, 0.18 mbar, 0.19 mbar or 0.2 mbar, for 5 to 15 hrs, for example, 5 hrs, 6 hrs, 7 hrs, 8 hrs, 9 hrs, 10 hrs, 11 hrs, 12 hrs, 13 hrs, 14 hrs or 15 hrs.

The present invention further provides use of the recombinant human HAase formulation in the formulation of a subcutaneous injection promoting agent.

The subcutaneous injection promoting agent includes at least the recombinant human HAase formulation.

The drug for subcutaneous injection preferably includes both or either of a drug carrier and/or a drug.

The drug carrier is conventional in the art, and preferably includes a glucose solution, an isotonic electrolyte solution, an alkaline solution, a hypertonic solution, dextran, a syrup substitute or a blood product. The drug carrier is deemed as an agent having therapeutic effect in clinical use in some cases.

The drug mentioned in the present invention is conventional in the art, and preferably includes chemotherapeutic agents, analgesics, anti-inflammatory agents, antimicrobial agents, anti-amoebic agents, trichomonacidal agents, anti-parkinsonism agent, anti-dysentery agents, antispasmodic agents, antidepressants, antirheumatic agents, antifungal agents, antihypertensive agents, antipyretics, antiparasitics, antihistamines, α-adrenergic agonists, α-blocking agents, anesthetics, bronchodilators, biocidal agents, bactericides, bacteriostatic agents, β -adrenergic blocking agents, calcium channel blocking agents, cardiovascular agents, contraceptives, decongestants, diuretics, sedatives, diagnostic agents, electrolyte agents, hypnotic agents, hormones, hyperglycemic agents, muscle relaxants, muscle contracting agents, ophthalmic agents, parasympathomimetics, psychostimulants, tranquilizers, sympathomimetics, tranquilizer, urinary agents, vaginal agents, antiviral agents, vitamin agents, non-steroidal anti-inflammatory agents, angiotensin converting enzyme inhibitors, polypeptides, proteins, nucleic acids, organic molecules, sleep promoters, insulin, cytokines, antibodies and monoclonal antibodies.

### 2. Drug combination

In a second aspect, the present invention provides a drug combination, which includes (1) a subcutaneous injection promoting agent containing the recombinant human HAase formulation as defined in the first aspect of the present invention; and (2) a drug for subcutaneous injection.

The drug combination described in the present invention is preferably a combination formulation or a kit consisting of separate packages of non-combination formulations. Preferably, it also contains a diluent or a reconstitution solvent. The diluent is preferably physiological saline. The reconstitution solvent is preferably water for injection or physiological saline.

### 2.1 Subcutaneous injection promoting agent

Preferably, the recombinant human HAase formulation includes 45 units/ml to 500000 units/ml recombinant human HAase.

The volume of the liquid formulation of recombinant human HAase or the volume after the lyophilized formulation is reconstituted is preferably 0.1 to 10.0 ml, for example, 0.2 ml, 0.5 ml, 1 ml, 1.5 ml, 2 ml, 2.5 ml, 3.0 ml, 3.5 ml, 4.0 ml, 4.5 ml, 5.0 ml, 5.5 ml, 6.0 ml, 7.0 ml, 8.0 ml, 9.0 ml, or 10.0 ml.

In some embodiments, 1 ml of the liquid formulation or the solution reconstituted with the lyophilized formulation of recombinant human HAase includes 45 units to 75000 units of recombinant human HAase, for example, 45 units, 100 units, 150 units, 200 units, 500 units, 1000 units, 1500 units, 2000 units, 2500 units, 3000 units, 4000 units, 4500 units, 5000 units, 6000 units, 6500 units, 7000 units, 7500 units, 8000 units, 8500 units, 9000 units, 10000 units, 15000 units, 20000 units, 21000 units, 22000 units, 23000 units, 24000 units, 25000 units, 26000 units, 27000 units, 28000 units, 29000 units, 30000 units, 31000 units, 32000 units, 33000 units, 34000 units, 35000 units, 36000 units, 37000 units, 38000 units, 39000 units, 40000 units, 41000 units, 42000 units, 43000 units, 44000 units, 45000 units, 46000 units, 47000 units, 48000 units, 49000 units, 50000 units, 55000 units, 60000 units, 65000 units, 70000 units or 75000 units of recombinant human HAase. Hereinafter, the volume of the recombinant human HAase formulation means the volume of the liquid formulation of recombinant human HAase or the solution obtained after the lyophilized formulation is reconstituted, and the concentration of the recombinant human HAase formulation means the concentration of the liquid formulation of recombinant human HAase or the solution obtained after the lyophilized formulation is reconstituted.

### 2.2 Drug for subcutaneous injection

Preferably, the drug combination further includes a drug for subcutaneous injection. Further preferably, the subcutaneous injection promoting agent is packaged into
(1) a separate package;
(2) a drug combination with a drug carrier for subcutaneous injection; and
(3) a drug combination with a drug for subcutaneous injection.

The drug carrier is conventional in the art, and preferably includes a glucose solution, an isotonic electrolyte solution, an alkaline solution, a hypertonic solution, dextran, a syrup substitute or a blood product. The drug carrier is deemed as an agent having therapeutic effect in clinical use in some cases.

The drug for subcutaneous injection may be a recombinant protein, a fusion protein, human albumin, immunoglobulin, a targeting antibody drug, a polypeptide, nanoparticles, viruses, cells or chemicals.

Preferably, the polypeptide can be insulin, glucagon-like peptide-1 (GLP-1) analog, leuprorelin, vasopressin, and bacitracin, etc.

Alternatively, the drug for subcutaneous injection is preferably a drug having anti-tumor effect.

The drug having anti-tumor effect can be conventional in the art, for example, one or more of oncolytic viruses, immune cells, CAR-T cells, TCR-T cells, anti-tumor drugs, antibody drugs targeting tumors, ADC antibody drugs, immune checkpoint inhibitors, nanoparticle drugs and corticosteroids.

The targets of the antibody drugs targeting tumors may be cell surface proteins, for example, AFP, av integrin, α4β7 integrin, BCMA, CD2, CD3, CD19, CD20, CD22, CD25, CD30, CD32, CD33, CD36, CD40, CD46, CD52, CD56, CD64, CD70, CD74, CD79, CD80, CD86, CD105, CD121, CD123, CD133, CD138, CD174, CD205, CD227, CD326, CD340, CEA, c-Met, Cripto, CA1X, Claudin18.2, ED-B, EGFR, EpCAM, EphA2, EphB2, FAP, FOLR1, GD2, Globo H, GPC3, GPNMB, HER-1, HER-2, HER-3, MAGE-A3, Mesothelin, MUC16, GPNMB, PSMA, TMEFF2, TAG-72, 5T4, ROR-1, Sca-1, SP, VEGF or WT1.

The targets of the anti-tumor drugs may be cytokines, for example, interleukins IL-1 to IL-13, tumor necrosis factors α and β, interferons α, β and γ, tumor growth factor β (TGF-β), colony-stimulating factor (CSF) or granulocyte-monocyte colony-stimulating factor (GMCSF). See Human Cytokines: Handbook for Basic & Clinical Research (Aggrawal et al eds, Blackwell Scientific, Boston, MA 1991).

The targets of the anti-tumor drugs can be hormones, enzymes, intracellular and intercellular messengers, for example, adenosine cyclase, guanosine cyclase or phospholipase C.

Preferably, the antibody drug targeting tumor is an antibody drug targeting human epidermal growth factor receptor 2 (Her-2), for example, trastuzumab, pertuzumab, ZW25, Margetuximab, DS8201 or ZW49.

Preferably, the antibody drug targeting tumor is an antibody drug targeting CD20, for example, rituximab.

Preferably, the antibody drug targeting tumor is an antibody drug targeting CD38, for example, daremumab, Isatuximab or MOR202.

Preferably, the effective ingredient of the anti-tumor drug includes gemcitabine, and paclitaxel, etc.

Preferably, the immune checkpoint inhibitors include anti-immune checkpoint antibodies, and the immune checkpoints include: CTLA-4, PD-1, PD-L1, TIM-3, LAG3, Siglec15, 4-1BB, GITR, OX40, CD40L, CD28, TIGIT, and VISTA.

The anti-immune checkpoint antibodies may be anti-PD-1 monoclonal antibody, anti-PD-L1 monoclonal antibody, anti-OX-40 monoclonal antibody, anti-CD47 monoclonal antibody, anti-CTLA-4 monoclonal antibody, anti-TIM-3 antibody, anti-LAG3 antibody, anti-Siglec15 antibody, anti-4-1BB antibody, anti-GITR antibody, anti-OX40 antibody, anti-CD40L antibody, anti-CD28 antibody, anti-TIGIT antibody, anti-VISTA antibody, and so on.

The targeting antibody drug may also be a non-tumor targeting antibody drug.

The non-tumor targeting antibody drug can be conventional in the art, and includes, for example, viral vector delivered drugs for gene therapy, monoclonal antibodies, bispecific antibodies, multispecific antibodies, chemicals, recombinant proteins and fusion proteins.

The targeting antibody drug can be one of Abagovomab, Abciximab, Actoxumab, Adalimumab, Adecatumumab, Afelimomab, Afutuzumab, Alacizumab pegol, ALD518, Alemtuzumab, Alirocumab, Altumomab pentetate, Amatuximab, Anatumomab mafenatox, Anrukinzumab, Apolizumab, Arcitumomab, Aselizumab, Atinumab, Atlizumab (=tocilizumab) , Atorolimumab, Bapineuzumab, Basiliximab, Bavituximab, Bectumomab, Belimumab, Benralizumab, Bertilimumab, Besilesomab, Bevacizumab, Bezlotoxumab, Biciromab, Bimagrumab, Bivatuzumabmertansine, Blinatumomab, Blosozumab, Brentuximab vedotin, Briakinumab, Brodalumab, Canakinumab, Cantuzumab mertansine, Cantuzumab ravtansine, Caplacizumab, Capromab pendetide, Carlumab, Catumaxomab, CC49, Cedelizumab, Certolizumab pegol, Cetuximab, Ch.14.18, Citatuzumab bogatox, Cixutumumab, Clazakizumab, Clenoliximab, Clivatuzumab tetraxetan, Conatumumab, Concizumab, Crenezumab, CR6261, Dacetuzumab, Daclizumab, Dalotuzumab, Daratumumab, Demcizumab, Denosumab, Detumomab, Dorlimomab aritox, Drozitumab, Duligotumab, Dupilumab, Dusigitumab, Ecromeximab, Eculizumab, Edobacomab, Edrecolomab, Efalizumab, Efungumab, Elotuzumab, Elsilimomab, Enavatuzumab, Enlimomab pegol, Enokizumab, Enoticumab, Ensituximab, Epitumomab cituxetan, Epratuzumab, Erlizumab, Ertumaxomab, Etaracizumab, Etrolizumab, Evolocumab, Exbivirumab, Fanolesomab, Faralimomab Farletuzumab, Fasinumab, FBTA05, Felvizumab, Fezakinumab, Ficlatuzumab, Figitumumab, Flanvotumab, Fontolizumab, Foralumab, Foravirumab, Fresolimumab, Fulranumab, Futuximab, Galiximab, Ganitumab, Gantenerumab, Gavilimomab, Gemtuzumab ozogamicin, Gevokizumab, Girentuximab, Glembatumumabvedotin, Golimumab, Gomiliximab, Ibalizumab, Ibritumomab tiuxetan, Icrucumab, Igovomab, Imciromab, Imgatuzumab, Inclacumab, Indatuximab ravtansine, Infliximab, Intetumumab, Inolimomab, Inotuzumab ozogamicin, Ipilimumab, Iratumumab, Itolizumab, Ixekizumab, Keliximab, Labetuzumab, Lampalizumab, Lebrikizumab, Lemalesomab, Lerdelimumab, Lexatumumab, Libivirumab, Ligelizumab, Lintuzumab, Lirilumab, Lodelcizumab, Lorvotuzumab mertansine, Lucatumumab, Lumiliximab, Mapatumumab, Maslimomab, Mavrilimumab, Matuzumab, Mepolizumab, Metelimumab, Milatuzumab, Minretumomab, Mitumomab, Mogamulizumab, Morolimumab, Motavizumab, Moxetumomab pasudotox, Muromonab-CD3, Nacolomab tafenatox, Namilumab, Naptumomab estafenatox, Narnatumab, Natalizumab, Nebacumab, Necitumumab, Nerelimomab, Nesvacumab, Nimotuzumab, Nivolumab, Nofetumomabmerpentan, Ocaratuzumab, Ocrelizumab, Odulimomab, Ofatumumab, Olaratumab, Olokizumab, Omalizumab, Onartuzumab, Oportuzumab monatox, Oregovomab, Orticumab, Otelixizumab, Oxelumab, Ozanezumab, Ozoralizumab, Pagibaximab, Palivizumab, Panitumumab, Panobacumab, Parsatuzumab, Pascolizumab, Pateclizumab, Patritumab, Pemtumomab, Perakizumab, Pertuzumab, Pexelizumab, Pidilizumab, Pinatuzumabvedotin, Pintumomab, Placulumab, Polatuzumab vedotin, Ponezumab, Priliximab, Pritoxaximab, Pritumumab, PRO 140, Quilizumab, Racotumomab, Radretumab, Rafivirumab, Ramucirumab, Ranibizumab,Raxibacumab, Regavirumab, Reslizumab, Rilotumumab, Rituximab, Robatumumab, Roledumab, Romosozumab, Rontalizumab, Rovelizumab, Ruplizumab, Samalizumab, Sarilumab, Satumomab pendetide, Secukinumab, Seribantumab, Setoxaximab, Sevirumab, Sibrotuzumab, Sifalimumab, Siltuximab, Simtuzumab, Siplizumab, Sirukumab, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tanezumab, Taplitumomab paptox, Tefibazumab, Telimomabaritox, Tenatumomab, Teneliximab, Teplizumab, Teprotumumab, Ticilimumab (=tremelimumab) , Tildrakizumab, Tigatuzumab, Tocilizumab (=atlizumab), Toralizumab, Tositumomab, Tralokinumab, Trastuzumab, TRBS07, Tregalizumab, Tucotuzumab celmoleukin, Tuvirumab, Ublituximab, Urelumab, Urtoxazumab, Ustekinumab, Vapaliximab, Vatelizumab, Vedolizumab, Veltuzumab, Vepalimomab, Vesencumab, Visilizumab, Volociximab, Vorsetuzumab mafodotin, Votumumab, Zalutumumab, Zanolimumab, Zatuximab, Ziralimumab or Zolimomab aritox.

### 2.3 Kit

The kit mentioned above in the present invention includes the recombinant human hydrate formulation as describe above, and also a drug for subcutaneous injection. The drug for subcutaneous injection includes both or either of a drug carrier and/or a drug.

In a first embodiment, the kit includes a single particular dose of the formulation containing 45 units/ml to 4500000 units/ml recombinant human HAase.

The container comprising the recombinant human HAase formulation is selected from a vial and a prefilled syringe, and preferably, a prefilled syringe.

The container comprising the recombinant human HAase formulation is selected from a vial and a prefilled syringe, and preferably, a vial.

Preferably, the recombinant human HAase formulation includes 150 units/ml to 500000 units/ml recombinant human HAase.

The volume of the recombinant human HAase formulation may be 0.2 ml, 0.5 ml, 1 ml, 1.5 ml, 2 ml, 2.50 ml, 5.00 ml, 10.00 ml, 15.00 ml, 20 ml, 30 ml, 40 ml or 50 ml.

In some embodiments, 1 ml of the recombinant human HAase formulation includes 150 units to 75000 units of recombinant human HAase, for example, 150 units, 200 units, 500 units, 1000 units, 1500 units, 2000 units, 2500 units, 3000 units, 4000 units, 4500 units, 5000 units, 6000 units, 6500 units, 7000 units, 7500 units, 8000 units, 8500 units, 9000 units, 10000 units, 15000 units, 20000 units, 21000 units, 22000 units, 23000 units, 24000 units, 25000 units, 26000 units, 27000 units, 28000 units, 29000 units, 30000 units, 31000 units, 32000 units, 33000 units, 34000 units, 35000 units, 36000 units, 37000 units, 38000 units, 39000 units, 40000 units, 41000 units, 42000 units, 43000 units, 44000 units, 45000 units, 46000 units, 47000 units, 48000 units, 49000 units, 50000 units, 55000 units, 60000 units, 65000 units, 70000 units or 75000 units of recombinant human HAase.

In a second embodiment, the kit includes two single-dose containers. A first container contains a fixed single dose of an antibody, and a second container contains a single particular dose of the formulation comprising 150 units/ml to 4500000 units/ml recombinant human HAase.

The first container is a vial, and the second container is a vial or a prefilled syringe.

The recombinant human HAase formulation preferably comprises 150 units/ml to 500000 units/ml recombinant human HAase; and further preferably 150 units/ml to 50000 units/ml recombinant human HAase.

In a third embodiment, the kit includes three single-dose containers. A first container contains a fixed single dose of pertuzumab, a second container contains a fixed single dose of trastuzumab, and a third container contains a single particular dose of the formulation comprising 150 units/ml to 4500000 units/ml recombinant human HAase.

The dose of pertuzumab is preferably 600 mg or 1200 mg, the dose of trastuzumab is preferably 600 mg, and the recombinant human HAase formulation preferably comprises 150 units/ml to 500000 units/ml recombinant human HAase, and further preferably 150 units/ml to 50000 units/ml recombinant human HAase. When pertuzumab and trastuzumab are used in combination, the dose of pertuzumab is 300 to 1500 mg, and preferably 600 mg or 1200 mg; and the dose of trastuzumab is 200 to 800 mg, and preferably 600 mg. The concentration of pertuzumab and/or trastuzumab may be 50 to 150 mg/ml; and preferably 60 to 120 mg/ml.

The first container is a vial, the second container is a vial, and the third container is a vial or a prefilled syringe.

In a fourth embodiment, the kit includes two single-dose containers. A first container contains a single particular dose of the formulation comprising 150 units/ml to 500000 units/ml recombinant human HAase, and a second container contains a fixed single dose of rituximab. The dose of rituximab is 700 to 1600 mg, and preferably 1400 mg or 1600 mg.

The concentration of rituximab may be 50 to 150 mg/ml; and preferably 60 to 120 mg/ml.

The recombinant human HAase formulation preferably comprises 150 units/ml to 500000 units/ml recombinant human HAase; and further preferably 150 units/ml to 50000 units/ml recombinant human HAase.

The first container is a vial or a prefilled syringe, and preferably a prefilled syringe; and the second container is a vial.

In a fifth embodiment, the kit includes two single-dose containers. A first container contains a single particular dose of the formulation comprising 150 units/ml to 500000 units/ml recombinant human HAase, and the second container contains a fixed single dose of trastuzumab. The dose of trastuzumab is 200 to 800 mg, and preferably 600 mg.

The concentration of trastuzumab may be 50 to 150 mg/ml; and preferably 60 to 120 mg/ml.

The recombinant human HAase formulation preferably comprises 150 units/ml to 100000 units/ml recombinant human HAase; and further preferably 150 units/ml to 50000 units/ml recombinant human HAase.

The first container is a vial or a prefilled syringe, and preferably a prefilled syringe; and the second container is a vial.

In a sixth embodiment, the kit includes two single-dose containers. A first container contains a single particular dose of the formulation comprising 150 units/ml to 500000 units/ml recombinant human HAase, and a second container contains a fixed single dose of immunoglobulin.

Immunoglobulin has a concentration of 5% to 20% (w/v).

The recombinant human HAase formulation preferably comprises 150 units/ml to 100000 units/ml recombinant human HAase; and further preferably 150 units/ml to 50000 units/ml recombinant human HAase.

The first container is a vial or a prefilled syringe, and preferably a prefilled syringe; and the second container is a vial.

In a seventh embodiment, the kit includes two single-dose containers. A first container contains a single particular dose of the formulation comprising 150 units/ml to 500000 units/ml recombinant human HAase, and a second container contains a fixed single dose of daratumumab and/or MOR202.

Daratumumab and/or MOR202 may have a concentration of 60-120 mg/ml.

The recombinant human HAase formulation preferably comprises 150 units/ml to 100000 units/ml recombinant human HAase; and further preferably 150 units/ml to 50000 units/ml recombinant human HAase.

The first container is a vial or a prefilled syringe, and preferably a prefilled syringe; and the second container is a vial.

In an eighth embodiment, the kit includes two single-dose containers. A first container contains a single particular dose of the formulation comprising 150 units/ml to 500000 units/ml recombinant human HAase, and a second container contains a fixed single dose of human albumin.

The human albumin has a concentration of 15% to 25% (w/v).

The recombinant human HAase formulation preferably comprises 150 units/ml to 100000 units/ml recombinant human HAase; and further preferably 150 units/ml to 50000 units/ml recombinant human HAase.

The first container is a vial or a prefilled syringe, and preferably a prefilled syringe; and the second container is a vial.

In a ninth embodiment, the kit includes two single-dose containers. A first container contains a single particular dose of the formulation comprising 150 units/ml to 500000 units/ml recombinant human HAase, and the second container contains a fixed single dose of Isatuximab.

Isatuximab may have a concentration of 60 to 120 mg/ml.

The recombinant human HAase formulation preferably comprises 150 units/ml to 100000 units/ml recombinant human HAase; and further preferably 150 units/ml to 50000 units/ml recombinant human HAase.

The first container is a vial or a prefilled syringe, and preferably a prefilled syringe; and the second container is a vial.

### 3. Method for treating diseases with the kit

In a third aspect, the present invention relates to a method for treating a disease by using the drug combination, particularly the kit, according to the second aspect, by administrating separately or in mixture.

In a preferred embodiment of the present invention, the steps of administrating the combination formulation to a subject in need of treatment include:
formulating the combination formulation, optionally added with or without a desired drug, into a solution intended to be injected, and then intradermally or subcutaneously administrating the solution to be injected to the subject in need of treatment.

In another preferred embodiment of the present invention, the steps of administrating the combination formulation to a subject in need of treatment include:
dissolving the combination formulation in a suitable drug carrier, to formulate a solution intended to be injected, and then intradermally or subcutaneously administrating the solution to be injected to the subject in need of treatment.

In another preferred embodiment of the present invention, the steps of administrating the kit to a subject in need of treatment include:
(a) formulating the subcutaneous injection promoting agent in a separate formulation package and the drug carrier in a separate formulation package, optionally mixed with/without a suitable drug, into a solution intended to be injected; and then intradermally or subcutaneously administrating the solution to be injected to the subject in need of treatment; or
(b) formulating the drug carrier in the kit, optionally mixed with/without a suitable drug, into a solution to be injected; intradermally or subcutaneously administrating the subcutaneous injection promoting agent in the kit to the subject in need of treatment, and then administrating the solution to be injected to the subject in need of treatment.

In another preferred embodiment of the present invention, the steps of administrating the kit to a subject in need of treatment include:
(a) mixing the subcutaneous injection promoting agent in a separate formulation package, the drug in a separate formulation package and a suitable drug carrier, formulating a solution intended to be injected, and then intradermally or subcutaneously administrating the solution to be injected to the subject in need of treatment; or
(b) mixing the drug in the kit with a suitable drug carrier, formulating a solution to be injected, intradermally or subcutaneously administrating the subcutaneous injection promoting agent in the kit to the subject in need of treatment, and then administrating the solution to be injected to the subject in need of treatment.

In another preferred embodiment of the present invention, the steps of administering the subcutaneous injection promoting agent to a subject in need of treatment include:
(a) formulating the subcutaneous injection promoting agent and a suitable drug carrier, optionally mixed with/without a suitable drug, into a solution intended to be injected, and then intradermally or subcutaneously administrating the solution to be injected to the subject in need of treatment; or
(b) formulating a suitable drug carrier, optionally mixed with/without a suitable drug, into a solution to be injected, intradermally or subcutaneously administrating the subcutaneous injection promoting agent to the subject in need of treatment, and then administering the solution to be injected to the subject in need of treatment.

The separate administration specifically includes the following steps:
(a) intradermally or subcutaneously administrating the recombinant human HAase formulation in the kit to the subject; and
(b) then administrating the drug for subcutaneous injection in the kit to the subject.

Steps (a) and (b) can be performed separately, simultaneously or alternatively.

When Steps (a) and (b) are performed separately, the time interval between Steps (a) and (b) is 0 to 24 hrs.

Preferably, the time interval is 0, at most 1 min, at most 2 min, at most 3 min, at most 4 min, at most 5 min, at most 6 min, at most 7 min, at most 8 min, at most 9 min, at most 10 min, at most 15 min, at most 20 min, at most 25 min, at most 30 min, at most 1 hr, at most 2 hrs, at most 3 hrs, at most 6 hrs, at most 12 hrs or at most 24 hrs.

In the case of sequential subcutaneous administration, the time interval between Steps (a) and (b) may be 0 min, 1 min, 2 min, 3 min, 4 min, 5 min, 6 min, 7 min, 8 min, 9 min, 10 min, 15 min, 20 min, 25 min, 30 min, 35 min, 40 min, 45 min, 50 min, 55 min or 60 min.

In the case of separate administration, simultaneous administration or sequential administration can be realized by a three-way valve.

Upon separate administration, the administration rate can be controlled by an injection pump or by gravity;

The recombinant human HAase formulation can be dosed at a rate of 0.1 to 2 ml/min.

The drug for subcutaneous injection can be injected at a rate of 5 ml/hr, 10 ml/hr, 30 ml/hr, 60 ml/hr, 120 ml/hr, 240 ml/hr or 300 ml/hr.

The administration in mixture includes mixing the recombinant human HAase formulation and the drug for subcutaneous injection in the kit and then subcutaneously administering to the subject. 1 ml of the formulation comprises 1000 units to 200000 units recombinant human HAase, for example, 1000 units, 2000 units, 3000 units, 4000 units, 5000 units, 6000 units, 7000 units, 8000 units, 9000 units, 10000 units, 15000 units, 20000 units, 25000 units, 30000 units, 35000 units, 40000 units, 45000 units, 50000 units, 55000 units, 60000 units, 65000 units, 70000 units, 75000 units, 80000 units, 85000 units, 90000 units, 95000 units, 100000 units, 105000 units, 110000 units, 115000 units, 120000 units, 125000 units, 130000 units, 135000 units, 140000 units, 145000 units, 150000 units, 155000 units, 160000 units, 165000 units, 170000 units, 175000 units, 180000 units, 185000 units, 190000 units, 195000 units or 200000 units recombinant human HAase.

The drug for subcutaneous injection intended to be mixed is in the form of a liquid or a dry powder.

Preferably, the recombinant human HAase formulation has an enzyme activity of 50000 units/ml, and a volume of 0.5 to 2 ml. When administered, the recombinant human HAase formulation was added to 10 to 15 ml of an injection containing 400 mg or 1600 mg rituximab, mixed uniformly and then injected subcutaneously.

The recombinant human HAase formulation can be administered directly and/or administered after dilution.

The diluent can be conventional in the art, for example, physiological saline.

The dilution ratio can be conventional in the art, for example, 1:10, 1:100, and 1:1000.

Use of the drug combination comprising trastuzumab, pertuzumab, and the recombinant human HAase formulation according to any of the foregoing embodiments in the formulation of drugs for treating HER2-positive tumors is provided. In a specific embodiment, the HER2-positive tumors include early breast cancer (EBC) or metastatic breast cancer (MBC), gastric cancer, gastroesophageal junction carcinoma, prostate cancer, non-small cell lung cancer, bladder cancer, ovarian cancer, colon cancer, esophageal cancer, and squamous cell carcinoma of the head and neck. In a preferred embodiment, the HER2-positive tumors are breast cancer and gastric cancer.

The drug combination further includes a chemical agent. The chemical agent is selected from the group consisting of taxane and anthracycline antibiotics. The taxane is paclitaxel or docetaxel. The anthracycline antibiotics can be daunorubicin, doxorubicin or epirubicin.

Use of the drug combination comprising rituximab, and the recombinant human HAase formulation according to any of the foregoing embodiments in the formulation of drugs for treating CD20-related tumors is provided. In a specific embodiment, the tumor includes recurrent or refractory low-grade or follicular, CD20 positive B-cell non-Hodgkin's lymphoma.

The drug combination further includes a chemical agent. The chemical agent is preferably cyclophosphamide, adriamycin, vincristine or prednisone.

Use of the drug combination comprising trastuzumab and the recombinant human HAase formulation according to any of the foregoing embodiments in the formulation of drugs for treating HER2-positive tumors is provided. In a specific embodiment, the HER2-positive tumors include early breast cancer (EBC) or metastatic breast cancer (MBC), gastric cancer, gastroesophageal junction carcinoma, prostate cancer, non-small cell lung cancer, bladder cancer, ovarian cancer, colon cancer, esophageal cancer, and squamous cell carcinoma of the head and neck. In a preferred embodiment, the HER2-positive tumors are breast cancer and gastric cancer.

The drug combination further includes a chemical agent. The chemical agent is selected from the group consisting of taxane and anthracycline antibiotics. The taxane is paclitaxel or docetaxel. The anthracycline antibiotics can be daunorubicin, doxorubicin or epirubicin.

Use of the drug combination comprising immunoglobulin and the recombinant human HAase formulation according to any of the foregoing embodiments in the formulation of drugs for treating diseases is provided. In a specific embodiment, the diseases are selected from immunodeficiency disease, ANCA-positive systemic necrotizing vasculitides, common variable immunodeficiency disease (CVID), primary immunodeficiency disease with antibody deficiency, congenital immunoglobulin deficiency, Wiskott-Aldrich syndrome, X-linked agammaglobulinemia (XLA), severe combined immunodeficiency disease (SCID), primary hypogammaglobulinemia, transient hypogammaglobulinemia of infancy, paraneoplastic cerebellar degeneration without antibodies, Alzheimer's disease, sepsis, cicatricial pemphigoid, B-cell tumor, acquired hypogammaglobulinemia secondary to hematological malignancies, Evans syndrome, acquired hypogammaglobulinemia secondary to hematological malignancies, acute disseminated encephalomyelitis, Kawasaki disease, chronic inflammatory demyelinating polyneuropathy (CIDP), multiple sclerosis, Guillain-Barre syndrome, idiopathic thrombocytopenic purpura, inflammatory myopathies, high-risk allogeneic hematopoietic stem cell transplantation, IgM paraproteinemic neuropathy, Lambert-Eaton myasthenic syndrome, toxic shock syndrome, multiple myeloma, multifocal motor neuropathy, myasthenia gravis, pemphigus foliaceus, Moersch-Woltmann syndrome, secondary hypogammaglobulinemia, specific antibody deficiency, autoimmune hemolytic anemia, bullous pemphigoid, fetomaternal/neonatal alloimmune thrombocytopenia (FMAIT/NAIT), hemophagocytic syndrome, renal transplantation, opsoclonus-myoclonus-ataxia, pemphigus vulgaris, post-transfusion purpura, toxic epidermal necrosis/StevenJohnson syndrome (TEN/SJS) , systemic lupus erythematosus, inflammatory myopathy, trauma, and bacterial, viral and fungal infections.

Preferably, the disease is caused by bacterial, viral or fungal infection. The bacterium, virus or fungus can be Haemophilus influenzae type B, Pseudomonas aeruginosa type A and B, Staphylococcus aureus, Group B streptococcus, Streptococcus pneumoniae type (1, 3, 4, 6, 7, 8, 9, 12, 14, 18, 19 and 23), Adenovirus type 2 and 5, herpes simplex virus type-1, herpes simplex virus type-2, cytomegalovirus, Epstein-Barr (EB) virus-viral capsid antigen (VCA), hepatitis A virus, hepatitis B virus, influenza A virus, measles virus, parainfluenza virus type (1, 2 and 3), poliovirus, varicella-zoster virus, Aspergillus or Candida albicans.

Use of the drug combination comprising daratumumab and the recombinant human HAase formulation according to any of the foregoing embodiments in the formulation of drugs for treating diseases is provided. In a specific embodiment, the disease is selected from multiple myeloma, light chain amyloidosis, leukemia, lymphoma, and solid tumor.

The drug combination further includes a chemical agent. The chemical agent is selected from glucocorticoids, proteasome inhibitors (PIs), cell cycle nonspecific agents, and immunomodulatory drugs (IMiDs) , for example, for example, survivin inhibitor, all-trans retinoic acid (ATRA), cyclophosphamide, doxorubicin, vincristine and prednisone (CHOP), dexamethasone, dehydrocortisone acetate, bortezomib, melphalan, lenalidomide, thalidomide or pomalidomide.

Use of the drug combination comprising human albumin and the recombinant human HAase formulation according to any of the foregoing embodiments in the formulation of drugs for treating diseases is provided. In a specific embodiment, the disease is selected from shock and cerebral edema caused by bleeding trauma and burn, edema or ascites caused by liver cirrhosis and kidney disease, and other critical illness, as well as hypoproteinemia.

Use of the drug combination comprising Isatuximab and the recombinant human HAase formulation according to any of the foregoing embodiments in the formulation of drugs for treating diseases is provided. In a specific embodiment, the disease is selected from multiple myeloma, light chain amyloidosis, leukemia, lymphoma, and solid tumor.

The drug combination further includes a chemical agent. The chemical agent is selected from glucocorticoids, proteasome inhibitors (PIs), cell cycle nonspecific agents, and immunomodulatory drugs (IMiDs) , for example, for example, survivin inhibitor, all-trans retinoic acid (ATRA), cyclophosphamide, doxorubicin, vincristine and prednisone (CHOP), dexamethasone, dehydrocortisone acetate, bortezomib, melphalan, lenalidomide, thalidomide or pomalidomide.

Preferably, the recombinant human HAase formulation has an enzyme activity of 500 units/ml, and a volume of 0.5 to 15 ml. Upon administration, the HAase formulation is administered by subcutaneous injection, and then rituximab injection is administered at a fixed dose of 1400 mg by subcutaneous injection. For patients with various CD20-positive B-cell malignant tumors, the treatment can be significantly simplified by subcutaneous administration, such that the original intravenous infusion over several hours can be changed to injection completed in 10 min, to improve the patient experience.

The direct administration of a liquid formulation or reconstituted solid formulation of recombinant human HAase of the present invention can promote the dissipation of local edema or hematoma after surgery and trauma, promote the infiltration of local anesthetics, relieve the pain at the injection site or accelerate the absorption of the drug solution administered by subcutaneous injection or intramuscular injection, alleviate the side effects of HA injection, promote the diffusion of the drug solution, exudate or blood accumulated locally in the eyes, promote the absorption of vitreous opacities, prevent atretoblepharia after chemical burn of conjunctiva and eliminate related inflammatory reactions, improve the reabsorption of the contrast agent barium sulfate (subcutaneous urethrography), and treat traumatic orbital hemorrhage and traumatic retinal edema.

The mode of administration for improving the reabsorption of the contrast agent barium sulfate is such that the recombinant human HAase formulation is injected subcutaneously at a dose of 50 to 5000 units at the scapula, and then the contrast agent is injected at the same site.

### 4. Package material and injection system

A fourth aspect of the present invention relates to a packaging material and injection system for the stable administration of the formulation and/or kit. The packaging material includes, but is not limited to, a vial, a syringe or a test tube. The injection system includes, but is not limited to, a syringe, an injection pump, an injection pen, a needle-free device or a subcutaneous patch delivery device.

The components in the injection device may be conventional, and includes a container, a sealing element and an injection needle.

The container includes, but is not limited to, a vial, a syringe or a test tube.

The material of the container may be conventional in the art, for example, glass or plastic.

The sealing element includes, but is not limited to, a sealing plug or a sealing ring.

The material of the sealing element may be conventional in the art, for example, rubber, plastic or a polymer material.

The injection needle includes, but is not limited to, a liquid injection needle, a single needle and a microneedle array.

The material of the injection needle may be conventional in the art, for example, metal, silicon, silicon dioxide, glass, nickel, titanium or a biodegradable polymer.

The liquid injection includes, but is not limited to, a liquid injection containing penicillin bottle, a liquid injection containing ampoule, or a prefilled injection system.

The ampoule for liquid injection may be a glass ampoule or a plastic ampoule.

The prefilled injection system can be conventional, for example, a prefilled syringe in the art.

Preferably, the container is a penicillin bottle made of neutral borosilicate glass with a specification of 0.1 to 1 ml.

The sealing element is a sealing plug made of halogenated butyl rubber.

The injection needle is a single needle or a microneedle array.

Preferably, the material of the single needle can be 304 or 316 stainless steel, with a specification of 30G, 24G, 27G or 29G (as shown in Table 1). The microneedle array can be made of 304 or 316 stainless steel, a biodegradable polymer, and includes nano-sized needles with a height of 10 to 2000 µm and a width of 10 to 50 µm.

| Specification | Inner diameter |
|---|---|
| 14G | 1.54 mm |
| 15G | 1.36 mm |
| 16G | 1.19 mm |
| 18G | 0.84 mm |
| 20G | 0.60 mm |
| 21G | 0.51 mm |
| 22G | 0.41 mm |
| 23G | 0.34 mm |
| 24G | 0.30 mm |
| 25G | 0.26 mm |
| 26G | 0.23 mm |
| 27G | 0.21 mm |
| 29G | 0.18 mm |
| 30G | 0.16 mm |
| 32G | 0.11 mm |
| 34G | 0.06 mm |

In one embodiment of the present invention, an article is provided, which comprises a drug formulation according to the second aspect of the present invention and instructions for use. The article further includes a container. The article may further include other materials desired from the commercial and user's point of view, including other buffers, diluents, filters, needles, syringes, infusion pumps and packaging inserts printed with instructions for use.

On the basis of common knowledge in the art, the above-mentioned preferred conditions can be combined at will, to obtain various preferred examples of the present invention.

The reagents and raw materials used in the present invention are all commercially available.

The present invention has the following positive progress. The stability of recombinant human HAase can be maintained at 2 to 8°C without special additives having the risk of contamination, such as human albumin, and the stability can be maintained at 95% or higher after storage for 12 months. The formulation enables a large volume of a drug solution to be administered by subcutaneous injection, so as to improve the treatment experience of patients and avoid the side effects related to intravenous infusion.

### Descriptions of Accompanying Drawings:

FIG. 1 shows the stability of liquid formulations of recombinant human HAase with various buffering agents.
FIG. 2 shows the stability of liquid formulations of recombinant human HAase with various concentrations of methionine.
FIG. 3 shows the stability of liquid formulations of recombinant human HAase with various concentrations of sodium chloride, trehalose, and sucrose.
FIG. 4 shows the stability of liquid formulations of recombinant human HAase with various concentrations of a surfactant.
FIG. 5 shows the stability of liquid formulations of recombinant human HAase at various concentrations.
FIG. 6 shows the stability of a liquid formulation of recombinant human HAase with one stabilizer being omitted.
FIG. 7 summarizes the stability of lyophilized formulations of recombinant human HAase with various buffering agents.
FIG. 8 summarizes the stability of lyophilized formulations of recombinant human HAase with various concentrations of methionine.
FIG. 9 summarizes the stability of lyophilized formulations of recombinant human HAase in the presence of various concentrations of sodium chloride, trehalose, and sucrose.
FIG. 10 summarizes the stability of lyophilized formulations of recombinant human HAase with various concentrations of mannitol.
FIG. 11 summarizes the stability of lyophilized formulations of recombinant human HAase with various concentrations of a surfactant.
FIG. 12 summarizes the stability of liquid formulations of recombinant human HAase at various concentrations.
FIG. 13 shows the bulges after the animals in each group receive continuous infusion of physiological saline.
FIG. 14 shows the bulges after the animals in each group receive continuous infusion of intralipid.
FIG. 15 shows pathological images of brain tissue, heart tissue and liver tissue after the injection of physiological saline and PH20, in which (a) shows the brain tissue in the physiological saline control group, (b) shows the brain tissue in the high-concentration PH20 formulation group, (c) shows the heart tissue in the physiological saline control group, (d) shows the heart tissue in the high-concentration PH20 formulation group, (e) shows the liver tissue in the physiological saline control group, and (f) shows the liver tissue in the high-concentration PH20 formulation group.
FIG. 16 shows pathological images of spleen tissue, lung tissue and kidney tissue after the injection of physiological saline and PH20, in which (a) shows the spleen tissue in the physiological saline control group, (b) shows the spleen tissue in the high-concentration PH20 formulation group, (c) shows the lung tissue in the physiological saline control group, (d) shows the lung tissue in the high-concentration PH20 formulation group, (e) shows the kidney tissue in the physiological saline control group, and (f) shows the kidney tissue in the high-concentration PH20 formulation group.

### Specific Embodiments

The present invention will be further described by way of examples below; however, the present invention is not limited thereto. In examples below where no specific conditions are given in the experimental methods, the experimental conditions are selected according to conventional methods and conditions, or according to the product instructions.

### Example 1: Formulation of recombinant human HAase

CHO cells were cultured in suspension in Dynamis serum-free medium, then subjected to fed-batch culture controlled by fluidically adding FeedC serum-free feed medium and then to shake-flask culture to gradually expand to a 30L-reactor scale.

After 3 to 4 days of culture, the amount of feed medium added to the bioreactor every day was 2% to 5% of the actual culture volume in the bioreactor. The culture temperature was controlled at 35°C to 37°C, and the pH was controlled at 7.0 by adding 10% Na₂CO₃ and CO₂. The aeration rate of the reactor was controlled at 0.015 to 0.15 vvm, the rotational speed was controlled at 80-150 rpm; and the dissolved oxygen was controlled at 20% to 40%. During cell culture, a sample was taken every day to monitor the temperature, pH, glucose concentration, lactic acid concentration, osmolarity and protein expression. When the viability of CHO cells was lower than 80% or the culture period reached 14 to 20 days, the culture was ended and recombinant human HAase was obtained.

The obtained supernatant containing recombinant human HAase was sequentially subjected to indepth filtration, anion chromatography, affinity chromatography, hydrophobic chromatography, cation chromatography, and ultrafiltration. Following this process, three batches of stock solutions were produced, and a recombinant human HAase stock solution with an SEC purity of over 95%, and HAase with an RP purity of over 85% were obtained, having an enzyme activity of 70000 units/mg or higher.

### Example 2: Detection and analysis of recombinant human hyaluronidase (HAase) formulation

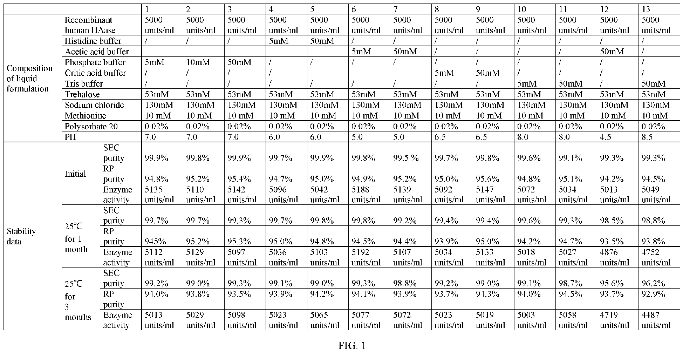

The medium in the recombinant human HAse stock solution obtained in Example 1 was replaced by liquid formulations of different compositions, and the recombinant human HAase was adjusted to a desired concentration. All liquid formulations were sterilized and filtered through a 0.22 µm low protein adsorption filter, and filled into a sterilized 5 ml glass vial under aseptic conditions. The vial was plugged with a fluororesin coated butyl rubber stopper and capped with an aluminum/plastic flip-off seal. The filling volume was 2 ml. These formulations were stored at different temperatures, and samples were taken at specified time intervals to investigate the stability of the formulations. The stability data of different liquid formulations were summarized. Samples were subjected to accelerated test at 25°C, and sampled at different times to analyze the HAase for SEC purity, RP purity and enzyme activity. The test results are shown in FIGs. 1-6.

As shown in FIG. 1, after standing for a period of time at 25°C, the SEC purity and enzyme activity of the recombinant human HAase at pH 4.5 and pH 8.5 decrease obviously. When the buffering agent is 5 mM and 50 mM phosphate buffer, histidine buffer, acetic acid buffer, citric acid buffer, or Tris buffer, the liquid formulations of recombinant human HAase have good stability at pH 5.0, 6.5 and 8.0.

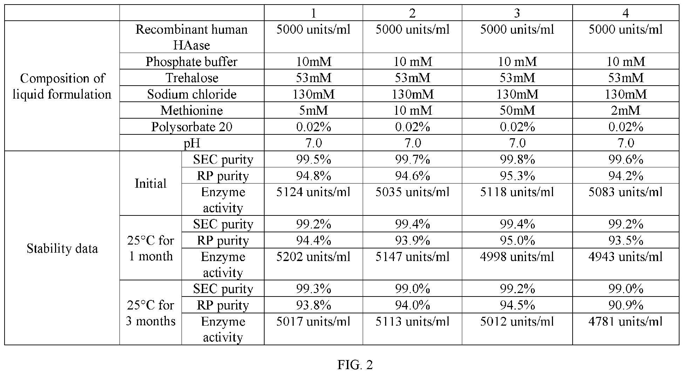

As shown in FIG. 2, after standing for a period of time at 25°C, the RP purity and enzyme activity of the recombinant human HAase in the presence of 2 mM methionine decrease obviously; and the liquid formulations of recombinant human HAase with 5, 10 and 50 mM methionine have good stability.

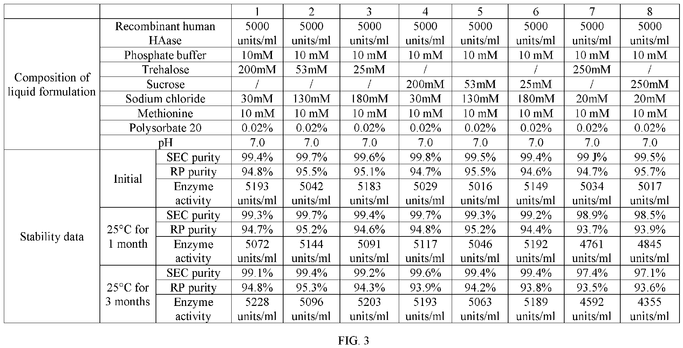

As shown in FIG. 3, after standing for a period of time at 25°C, with a stabilizer that comprises 20 mM sodium chloride, 250 mM trehalose and 20 mM sodium chloride, or 250 mM sucrose, the SEC purity and enzyme activity of the recombinant human HAase decrease obviously; and with a stabilizer that comprises 30, 130 or 180 mM sodium chloride, and/or 25, 53 or 200 mM trehalose, and/or 25, 53 or 200 mM sucrose, the liquid formulations of recombinant human HAase have good stability.

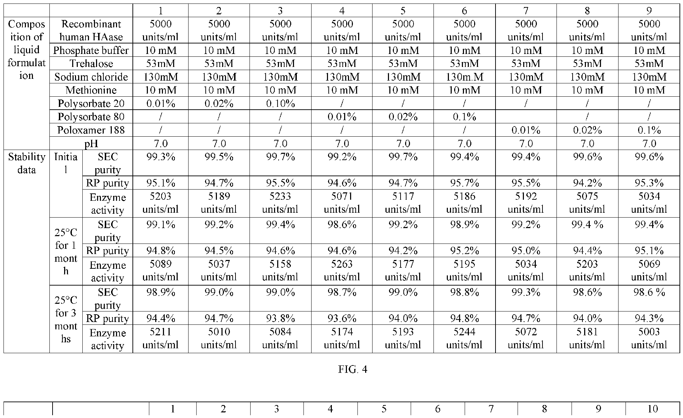

As shown in FIG. 4, with a surfactant that is polysorbate 20 having a concentration of 0.01 to 0.1%, polysorbate 80 having a concentration of 0.01 to 0.1%, or Poloxamer188 having a concentration of 0.01 to 0.1%, the liquid formulations of recombinant human HAase have good stability.

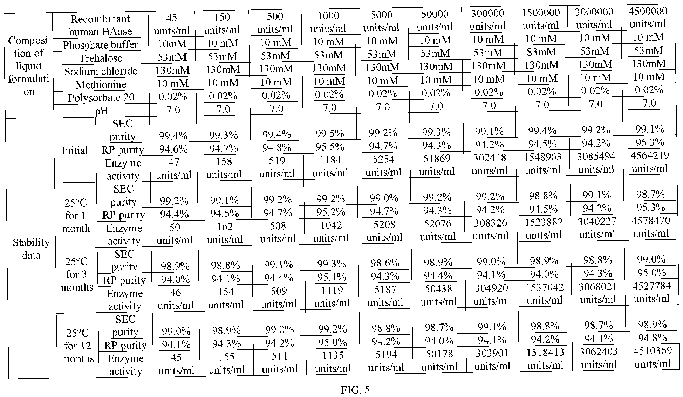

As shown in FIG. 5, the recombinant human HAase activity in the liquid formulation of recombinant human HAase in this example is stable at 150, 500, 1, 000, 5, 000, 50, 000, 300, 000, 1, 500, 000, 3, 000, 000 and 4, 500, 000 units/ml, and the enzyme activity can still be maintained at 95% or higher after storage for 12 months at 2 to 8°C.

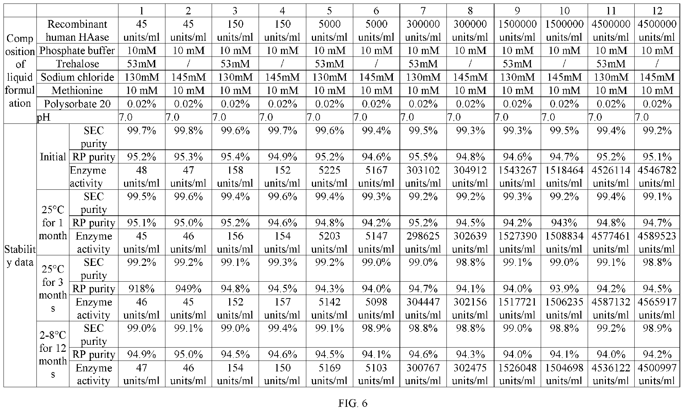

As shown in FIG. 6, with increasing ingredients in the protein formulation, the uncontrollable risks increase. The stability study of HAase liquid formulation with one stabilizer being omitted shows that when a sugar stabilizer is removed from a liquid formulation of recombinant human HAase, liquid formulations with different concentrations of recombinant human HAase still have good stability, and the enzyme activity of each formulation is maintained at 95% or higher after storage for 12 months at 2 to 8°C.

### Example 3: Study of liquid formulation of recombinant human HAase

The medium in the recombinant human HAse stock solution was replaced by a liquid formulation with a different composition, and the recombinant human HAase was adjusted to a desired concentration. All liquid formulations were sterilized and filtered through a 0.22 µm low protein adsorption filter, and filled into a sterilized 5 ml glass vial under aseptic conditions. The vial was plugged with a fluororesin coated butyl rubber stopper and capped with an aluminum/plastic flip-off seal. The filling volume was 2 ml. These formulations were stored at different temperatures, and samples were taken at specified time intervals to investigate the stability of the formulations. The stability data of different liquid formulations were summarized. Samples were subjected to accelerated test at 25°C, and sampled at different times to analyze the HAase for SEC purity, RP purity and enzyme activity. The test results are shown in FIGs. 1-6.

As shown in FIG. 1, after standing for a period of time at 25°C, the SEC purity and enzyme activity of the recombinant human HAase at pH 4.5 and pH 8.5 decrease obviously. When the buffering agent is 5 mM and 50 mM phosphate buffer, histidine buffer, acetic acid buffer, citric acid buffer, or Tris buffer, the liquid formulations of recombinant human HAase have good stability at pH 5.0, 6.5 and 8.0.

As shown in FIG. 2, after standing for a period of time at 25°C, the RP purity and enzyme activity of the recombinant human HAase in the presence of 2 mM methionine decrease obviously; and the liquid formulations of recombinant human HAase with 5, 10 and 50 mM methionine have good stability.

As shown in FIG. 3, after standing for a period of time at 25°C, with a stabilizer that comprises 20 mM sodium chloride, 250 mM trehalose and 20 mM sodium chloride, or 250 mM sucrose, the SEC purity and enzyme activity of the recombinant human HAase decrease obviously; and with a stabilizer that comprises 30, 130 or 180 mM sodium chloride, and/or 25, 53 or 200 mM trehalose, and/or 25, 53 or 200 mM sucrose, the liquid formulations of recombinant human HAase have good stability.

As shown in FIG. 4, with a surfactant that is polysorbate 20 having a concentration of 0.01 to 0.1%, polysorbate 80 having a concentration of 0.01 to 0.1%, or Poloxamer188 having a concentration of 0.01 to 0.1%, the liquid formulations of recombinant human HAase have good stability.

As shown in FIG. 5, the recombinant human HAase activity in the liquid formulation of recombinant human HAase in this example is stable at 150, 500, 1, 000, 5, 000, 50, 000, 300, 000, 1, 500, 000, 3, 000, 000 and 4, 500, 000 units/ml, and the enzyme activity can still be maintained at 95% or higher after storage for 12 months at 2 to 8°C.

As shown in FIG. 6, with increasing ingredients in the protein formulation, the uncontrollable risks increase. The stability study of HAase liquid formulation with one stabilizer being omitted shows that when a sugar stabilizer is removed from a liquid formulation of recombinant human HAase, liquid formulations with different concentrations of recombinant human HAase still have good stability, and the enzyme activity of each formulation is maintained at 95% or higher after storage for 12 months at 2 to 8°C.

### Example 4: Study of lyophilized formulation of recombinant human HAase

Various liquid formulations of recombinant human HAase were prepared, then lyophilized, and lyophilized according to Table 1, and then the stability of various lyophilized formulations was studied.

**Table 1**

| Operation steps | Set plate temperature (° C) | Holding time (hr) | Set vacuum level (mbar) |
|---|---|---|---|
| (1) Pre-freezing | -5 | 1.0 | |
| (2) Pre-freezing | -40 | 3 | |
| (3) Drying | -25 | 19.5 | 15 |
| (4) Drying | -20 | 1.5 | 15 |
| (5) Drying | 0 | 2.0 | 15 |
| (6) Drying | 25 | 8.5 | 15 |

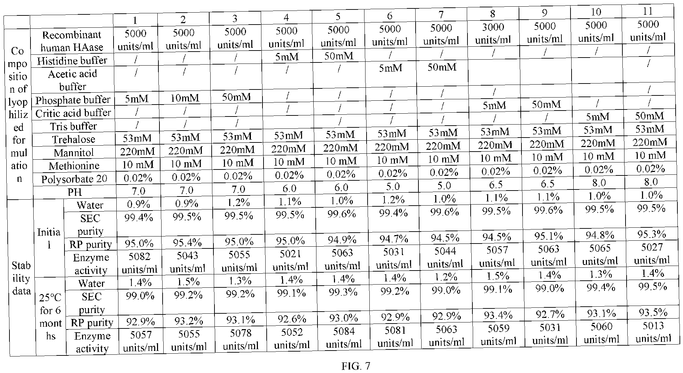

As shown in FIG. 7, when the buffering agent is 5 mM and 50 mM phosphate buffer, histidine buffer, acetic acid buffer, citric acid buffer, or Tris buffer, the lyophilized formulations of recombinant human HAase have good stability at pH 5.0, 6.0, 6.5, 7.0 and 8.0.

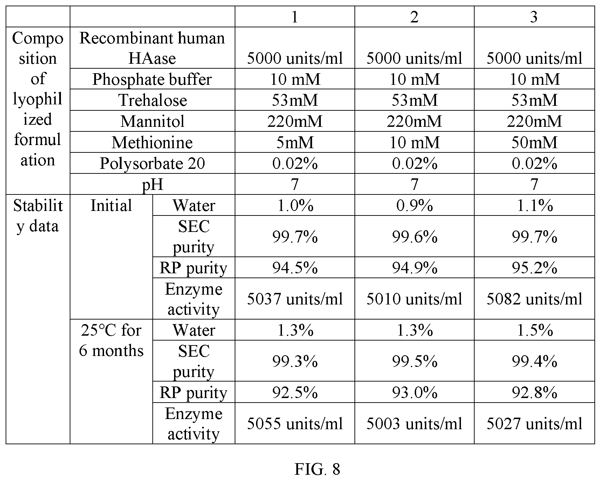

As shown in FIG. 8, the lyophilized formulations of recombinant human HAase with methionine having a concentration of 5, 10 or 50 mM have good stability.

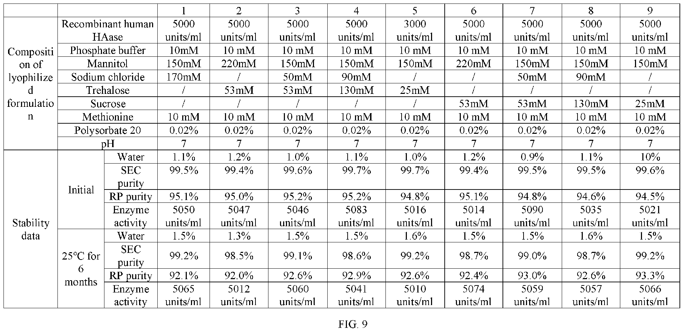

As shown in FIG. 9, with a stabilizer that comprises 50 to 170 mM sodium chloride, 25 to 130 mM trehalose, and/or 25 to 130 mM sucrose, the lyophilized formulations of recombinant human HAase have good stability.

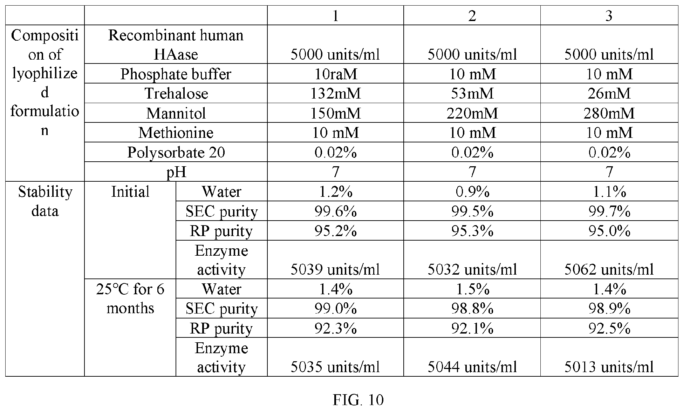

As shown in FIG. 10, with a cryoprotectant that comprises 150 to 280 mM mannitol, the lyophilized formulations of recombinant human HAase have good stability.

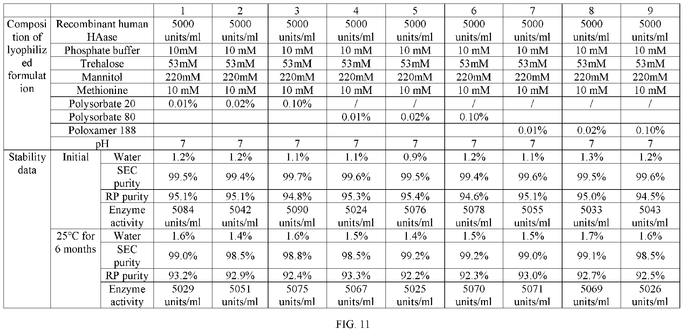

As shown in FIG. 11, with a surfactant that is 0.01% to 0.1% polysorbate 20, 0.01% to 0.1% polysorbate 80, or 0.01% to 0.1% Poloxamer 188, the lyophilized formulations of recombinant human HAase have good stability.

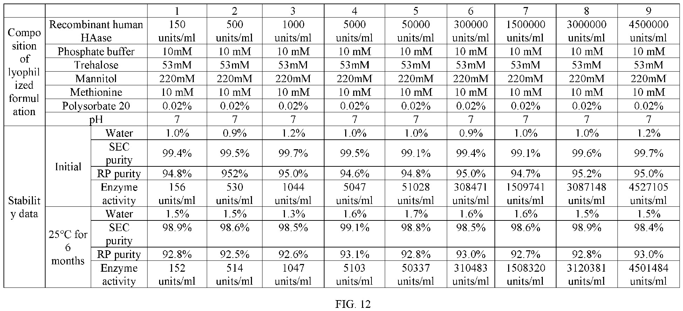

As shown in FIG. 12, lyophilized formulations of recombinant human HAase containing 150 to 4500000 units/ml recombinant human HAase have good stability.

### Example 5: Subcutaneous injection of large volume of drug solution into miniature pigs

The purpose of this experiment was to observe the effect of subcutaneous injection of an HAase liquid formulation on subcutaneous injection of physiological saline in miniature pigs. The infusion pressure was recorded, the degree of skin swelling and red spot at the injection site were observed, and the time required for swelling to return to normal was recorded.

Eight Bama miniature pigs were randomly divided into 2 groups according to their weight, namely, 1- negative control group (administered with physiological saline) and 2-recombinant human HAase group (150 units/site), with 4 pigs in each group. The injection pressure was measured by a multichannel physiological signal recorder. After the HAase formulation was administered, 150 mL of physiological saline was continuously injected at a rate of 1200 ml/h During injection, the degree of skin swelling and red spot at the injection site were observed, and the time required for swelling to return to normal was recorded.

In the negative control group, after the animals are injected with 150 mL physiological saline, obvious bulges are formed at the injection site, the swelling score is 4.0±0.0, and the time required for swelling to return to normal is 125.5±8.1 min. After the recombinant human HAase formulation is injected, the swelling degree is lower than that in the negative control group, and the swelling score is 2.3±0.5, so the swelling degree is significantly decreased (*P*≤0.001). The time required for swelling to return to normal is 33.3±8.4 min in the recombinant human HAase formulation group, which is significantly lower than that in the negative control group (P<0.001).

In the negative control group, slight to moderate red spot is observed in several animals after injection. In the recombinant human HAase formulation group, extremely slight red spot can be observed in several animals after injection. There is no statistical difference in red spot score between the two groups (*P*≥0.05).

The baseline values of pressure before subcutaneous injection of physiological saline to animals in the negative control group and the recombinant human HAase formulation group are close, and respectively 1.5±5.5 mmHg, 5.1±3.2 mmHg. With increasing volume of injection, the injection pressure in the negative control group increases rapidly. The injection pressure at an injection volume of 25 mL, 50 mL, 100 mL and 150 mL is respectively 270.5±153.1 mmHg, 235.1±119.4 mmHg, 215.9±95.1 mmHg, 213.7±90.5 mmHg. In the recombinant human HAase formulation group, the injection pressure at corresponding injection volume is 171.6±111.7 mmHg, 164.5±107.2 mmHg, 152.2±88.3 mmHg, and 141.5±77.7 mmHg respectively, which are all lower than those in the negative control group, and have no statistical difference (*P*≥0.05).

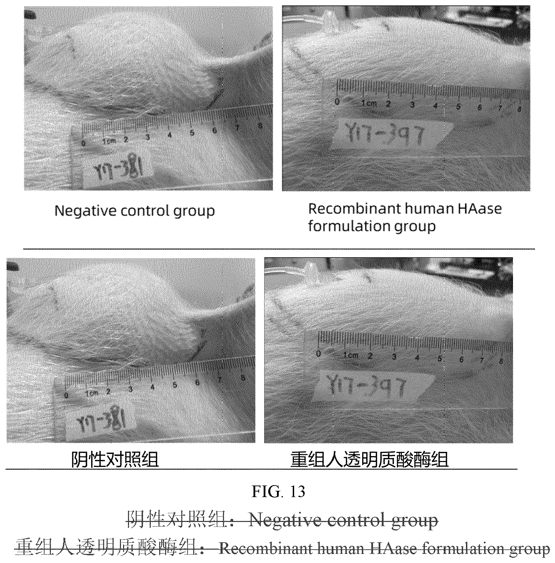

Under the experimental conditions in this example, the subcutaneous injection of the test HAase liquid formulation in miniature pigs can reduce the subcutaneous injection pressure, the degree of skin swelling and deformation after injection and the time required to return to normal, and promote the rapid diffusion and absorption of a liquid injected (FIG. 13).

### Example 6: Subcutaneous injection of large volume of drug solution into miniature pigs

The purpose of this experiment was to observe the effect of subcutaneous injection of an HAase liquid formulation on subcutaneous injection of fat emulsion injection in miniature pigs. The infusion pressure was recorded, the degree of skin swelling and red spot at the injection site were observed, and the time required for swelling to return to normal was recorded.

Eight Bama miniature pigs were randomly divided into 2 groups according to their weight, namely, 1- negative control group (administered with physiological saline) and 2-recombinant human HAase group (150 units/site), with 4 pigs in each group. The injection pressure was measured by a multichannel physiological signal recorder. After the HAase formulation was administered, 50 mL of fat emulsion injection was continuously injected at a rate of 300 ml/h.. During injection, the degree of skin swelling and red spots at the injection site were observed, and the time required for swelling to return to normal was recorded.

The results show that in the negative control group, after the animals are injected with 50 mL fat emulsion injection, obvious bulges are formed at the injection site, the swelling score is 4.0±0.0, and the time required for swelling to return to normal is 125.0±33.9 min. After the recombinant human HAase formulation is injected, the swelling degree is lower than that in the negative control group, and the swelling score is 1.8±0.5, so the swelling degree is significantly decreased (*P*≤0.001). The time required for swelling to return to normal is 22.1±6.8 min in the recombinant human HAase formulation group, which is significantly lower than that in the negative control group (*P*≤0.001).

In the negative control group, slight to moderate red spot is observed in several animals after injection. In the recombinant human HAase formulation group, extremely slight red spot can be observed in several animals after injection. There is no statistical difference in red spot score between the two groups (*P*≥0.05).

The baseline values of pressure before subcutaneous injection of fat emulsion injection to animals in the negative control group and the recombinant human HAase formulation group are close, and respectively 3.1±2.7 mmHg and 1.8±2.4 mmHg. With increasing volume of injection, the injection pressure in the negative control group increases rapidly. The injection pressure at an injection volume of 5 mL, 10 mL, 20 mL, 30 mL and 50 mL is respectively 65.2±19.6 mmHg, 84.9±25.9 mmHg, 114.6±27.1 mmHg, 114.8±40.0 mmHg and 128.6±48.2 mmHg. In the recombinant human HAase formulation group, the injection pressure at corresponding injection volume is 32.9±13.8 mmHg and 35.7±20.7 mmHg, 36.1±18.1 mmHg, 35.5±12.2 mmHg and 32.0±11.2 mmHg respectively, which are all lower than those in the negative control group, and have no statistical difference (*P*≥0.05).

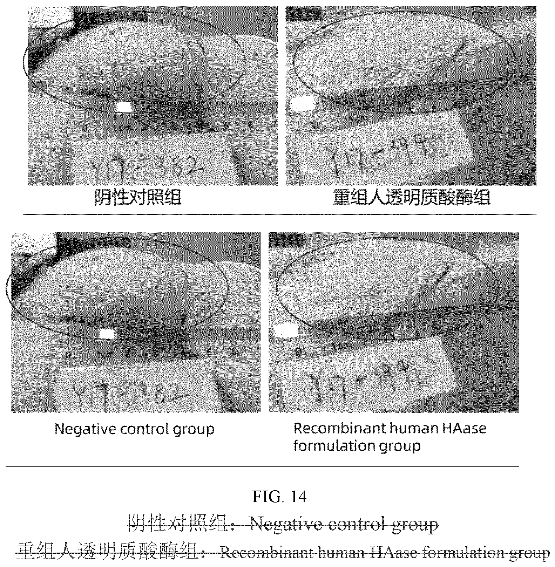

Under the experimental conditions in this example, the subcutaneous injection of the test HAase liquid formulation in miniature pigs can reduce the subcutaneous injection pressure, the degree of skin swelling and deformation after injection and the time required to return to normal, and promote the rapid diffusion and absorption of a liquid injected (FIG. 14).

### Example 7: Liquid formulation of recombinant human HAase improves the absorption rate of subcutaneously injected high concentration protein solution in nude mice

The purpose of this experiment was to observe the effect of subcutaneous injection of an HAase liquid formulation on pressurized subcutaneous infusion of high concentration human albumin solution in nude mice at the dorsal side. The injection time was recorded and the injection rate was calculated.

36 female BALB/c nude mice were randomly divided into 2 groups according to the weight, namely: 1-positive control group (50 units/site) and 2-HAase liquid formulation group (50 units/site), with 18 animals in each group. After the animals were anesthetized, 2 injection sites were drawn symmetrically on the dorsal side with a MARK pen. The HAase liquid formulation or positive control solution was injected subcutaneously at the right dorsal side, and a corresponding volume of physiological saline was injected subcutaneously at the left dorsal side as a self-negative control. One end of a PE tube with an inner diameter of 0. 76 mm was connected with a 1 mL syringe containing 50 mg/mL human albumin solution, and the other end was connected with a 0.55 mm needle. 600 µL of human albumin was subcutaneously injected at the administration site under a water pressure of 20 cm, 30 cm and 40 cm (6 animals in each group). The injection time t (min) was recorded and the injection rate v (µL/min) was calculated.

In the HAase liquid formulation group, the injection rates of human albumin solution subcutaneously injected in animals at the left dorsal side (administered with physiological saline) under a water pressure of 20 cm, 30 cm and 40 cm are respectively 54.9±6.8 µL/min, 113.0±20.1 µL/min and 201.5±48.3 µL/min; and the injection rates of human albumin solution subcutaneously injected in animals at the right dorsal side (administered with HAase liquid formulation) under various corresponding water pressures are respectively 326.1±38.4 µL/min, 749.3±40.6 µL/min and 1140.5±160.4 µL/min. Compared with the left self-negative control group, the injection rate of human albumin under various pressures are obviously improved (P≤0.01).

Under the experimental conditions in this example, the HAase liquid formulation can significantly increase the rate of subcutaneous injection of high concentration human albumin solution in nude mice.

### Example 8: Liquid formulation of recombinant human HAase improves the absorption rate of subcutaneously injected rituximab in nude mice

The purpose of this experiment was to observe the effect of subcutaneous injection of an HAase liquid formulation on pressurized subcutaneous infusion of rituximab (120 mg/ml) in nude mice at the dorsal side. The infusion time was recorded and the injection rate was calculated.

36 female BALB/c nude mice were randomly divided into 3 groups according to the weight, namely: 1-negative control group (physiological saline + rituximab), 2-HAase liquid formulation group (50 units/site, where the HAase liquid formulation was administered before rituximab is injected), and 3-HAase liquid formulation + rituximab mixture group, with 12 animals in each group. Before administration in the 3 groups, 300000 units/ml recombinant human HAase solution was added to the rituximab solution at a ratio of 1:60, mixed uniformly, and administered. After the animals were anesthetized, 2 injection sites were drawn symmetrically on the dorsal side with a MARK pen. The HAase liquid formulation or positive control solution was injected subcutaneously at the right dorsal side, and a corresponding volume of physiological saline was injected subcutaneously at the left dorsal side as a self-negative control. One end of a PE tube with an inner diameter of 0.76 mm was connected with a 1 mL syringe containing 120 mg/mL rituximab solution, and the other end was connected with a 0.55 mm needle. 600 µL of rituximab was subcutaneously injected at the administration site under a water pressure of 20 cm, 30 cm and 40 cm (6 animals in each group). The injection time t (min) was recorded and the injection rate v (µL/min) was calculated.

In the HAase liquid formulation group, the injection rates of rituximab solution subcutaneously injected in animals at the left dorsal side (administered with physiological saline) under a water pressure of 20 cm, 30 cm and 40 cm are respectively 44.5±3.4 µL/min, 92.1±15.2 µL/min and 182.5±38.1 µL/min; and the injection rates of rituximab solution subcutaneously injected in animals at the right dorsal side (administered with HAase liquid formulation) under various corresponding water pressures are respectively 305.1±39.5 µL/min, 734.2±51.2 µL/min and 1251.3±171.7 µL/min. Compared with the left self-negative control group, the injection rate of rituximab solution under various pressures are obviously improved (P≤0.01).

Under the experimental conditions in this example, the HAase liquid formulation can significantly increase the rate of subcutaneous injection of high concentration rituximab solution in nude mice.

### Example 9: Liquid formulation of recombinant human HAase improves the absorption rate of subcutaneously injected trastuzumab in nude mice

The purpose of this experiment was to observe the effect of subcutaneous injection of an HAase liquid formulation on pressurized subcutaneous infusion of trastuzumab (120 mg/ml) in nude mice at the dorsal side. The infusion time was recorded and the injection rate was calculated.

36 female BALB/c nude mice were randomly divided into 3 groups according to the weight, namely: 1-negative control group (physiological saline + trastuzumab), 2-HAase liquid formulation group (50 units/site, where the HAase liquid formulation was administered before trastuzumab is injected), and 3-HAase liquid formulation + trastuzumab mixture group, with 12 animals in each group. Before administration in the 3 groups, 300000 units/ml recombinant human HAase solution was added to the trastuzumab solution at a ratio of 1:60, mixed uniformly, and administered. After the animals were anesthetized, 2 injection sites were drawn symmetrically on the dorsal side with a MARK pen. The HAase liquid formulation or positive control solution was injected subcutaneously at the right dorsal side, and a corresponding volume of physiological saline was injected subcutaneously at the left dorsal side as a self-negative control. One end of a PE tube with an inner diameter of 0.76 mm was connected with a 1 mL syringe containing 120 mg/mL trastuzumab solution, and the other end was connected with a 0.55 mm needle. 600 µL of trastuzumab was subcutaneously injected at the administration site under a water pressure of 20 cm, 30 cm and 40 cm (6 animals in each group). The injection time t (min) was recorded and the injection rate v (µL/min) was calculated.

In the HAase liquid formulation group, the injection rates of trastuzumab solution subcutaneously injected in animals at the left dorsal side (administered with physiological saline) under a water pressure of 20 cm, 30 cm and 40 cm are respectively 34.5±7.4 µL/min, 111.8±19.6 µL/min and 192.5±54.5 µL/min; and the injection rates of trastuzumab solution subcutaneously injected in animals at the right dorsal side (administered with HAase liquid formulation) under various corresponding water pressures are respectively 284.4±52.4 µL/min, 851.2±60.3 µL/min and 1004.1±187.1 µL/min. Compared with the left self-negative control group, the injection rate of trastuzumab solution under various pressures are obviously improved (P≤0.01).

Under the experimental conditions in this example, the HAase liquid formulation can significantly increase the rate of subcutaneous injection of high concentration trastuzumab solution in nude mice.

### Example 10: Safety evaluation of high-concentration liquid formulation of recombinant human HAase

The toxicity test of repeated administration by subcutaneous injection was carried out in Cynomolgus monkeys to evaluate the safety of high-concentration PH20 formulation.

20 Cynomolgus monkeys were assigned to a physiological saline control group and a high-concentration PH20 formulation group according to their weight, with 5 monkeys/gender in each group. The animals in the high-concentration PH20 formulation group were administered at a dosage of 0.04 mg/kg (equivalent to 12000 units/kg), once a day, for consecutive 28 days. According to the weight before administration, the dosage for each animal was determined and administered subcutaneously at the medial sides of hind limbs. The animals in the physiological saline control group were given physiological saline subcutaneously every day, and the other experimental conditions were the same as those in the high-concentration PH20 formulation group.

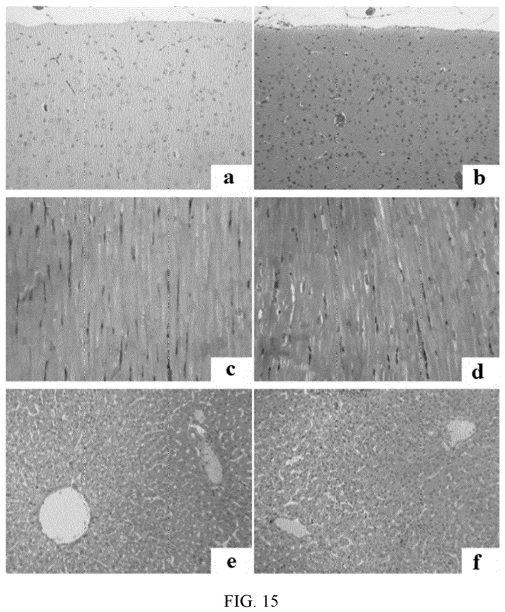

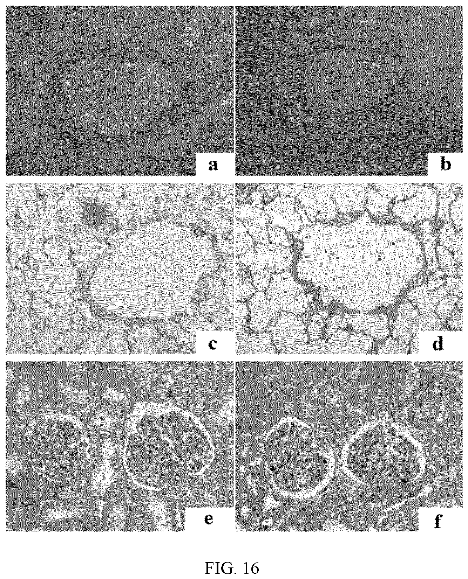

No dying or death of animals was observed during the administration period. All the animals were euthanized and systematically anatomized on the following day (D29) after the administration period, and the main organs were histopathologically examined. The results show that in the physiological saline control group and the high-concentration PH20 formulation group, the gross anatomy and microscopic examination results show no abnormal pathological changes related to injection in animals. The histopathological images of main organs are shown in FIGs. 15-16.

All animals were observed once each day before administration during the administration period and the day after the last administration to observe the local occurrence of swelling, fever, edema, red spot, ulceration and other changes at the injection site. The results show that no abnormalities are clinically observed locally at the administration site in all the animals during the administration period; the gross anatomy on the following day (D29) after the last administration shows no abnormal physiological changes associated with injection locally at the administration site in all the animals; and compared with the physiological saline control group, no irritation reactions related to the high-concentration PH20 formulation are microscopically observed at the injection site in animals in the high-concentration PH20 formulation group.

In addition, compared with the control group, the hematological indexes of all the animals show no obvious abnormalities. For example, for the hematological indexes on the following day (D29) after the last administration, WBC (x 10⁹/L) in the physiological saline control group and the high-concentration PH20 formulation group is respectively 9.356±1.615 and 9.174±1.569, Neut (%) is respectively 31.92±7.52 and 37.70±14.89, Lymph (%) is respectively 58.94±7.08 and 55.06±15.13, RBC (x 10¹²/L) is respectively 5.654±0.295 and 5.368±0.364, HGB (g/L) is respectively 128.4±6.7 and 123.2±6.0, and PLT (x 10⁹/L) is respectively 341.8±97.5 and 346.8±62.3.

With respect to the blood biochemical indexes of the animals, no abnormal changes are observed in the physiological saline control group and high-concentration PH20 formulation group. For example, for the blood biochemical indexes on the following day (D29) after the last administration, ALT(U/L) in the physiological saline control group and the high-concentration PH20 formulation group is respectively 33.5±4.9 and 35.6±5.1, AST (U/L) is respectively 63.4±17.9 and 68.0±6.3, ALP(U/L) is respectively 405.6±81.7 and 405.6±89.5, TBil (µmol/L) is respectively 2.268±0.516 and 2.182±0.698, LDH (U/L) is respectively 637.4±131.9 and 658.8±111.5, Cre (µmol/L) is respectively 61.6±10.7 and 62.6±9.5, TG (mmol/L) is respectively 0.478±0.089 and 0.468±0.054, TP(g/L) is respectively 74.68±0.77 and 73.56±3.69, and Glu (mmol/L) is respectively 3.718±0.765 and 3.758±0.416.

Therefore, subcutaneous injection of high-concentration PH20 formulation is safe and nonirritating locally to the administration site.

Detailed methods of the present invention are described by way of examples above, but the present invention is not limited thereto. That is, the present invention can be implemented otherwise with no need to have to rely on the above-mentioned detailed methods. It is to be understood by those skilled in the art that any improvement made to the present invention, equivalent replacement of raw materials, addition of auxiliary components, and selection of specific methods, etc. fall within the scope of protection and disclosure of the present invention.

## Claims

1. A recombinant human hyaluronidase (HAase) formulation, comprising a recombinant human HAase, a buffering agent, a stabilizer and a nonionic surfactant, wherein
the recombinant human HAase has an enzyme activity of 45 units/ml to 4500000 units/ml;
the buffering agent has a concentration of 5 to 50 mM;
the stabilizer has a concentration of 1 to 500 mM, and the stabilizer is one or more selected from trehalose, sucrose, mannitol, sodium chloride, and methionine;
the nonionic surfactant has a concentration of 0.01% to 0.1% (w/v); and
the formulation has a pH of 5.0 to 8.0.

2. The formulation according to claim 1, wherein the recombinant human HAase has an enzyme activity of 45 units/ml to 3000000 units/ml, preferably 45 units/ml to 1500000 units/ml, and further preferably 45 units/ml to 300000 units/ml;
and/or the recombinant human HAase comprises an amino acid sequence as shown in SEQ ID NO. 1; and preferably the recombinant human HAase has an amino acid sequence as shown in SEQ ID NO. 2;
and/or the stabilizer has a concentration of 100 to 400 mM, and preferably 150 to 350 mM, for example, 155 mM, 188 mM, 193 mM, 215 mM, 233 mM, 240 mM, 263 mM, 283 mM, 292 mM, 316 mM or 330 mM.

3. The formulation according to claim 1, wherein
the trehalose or sucrose has a concentration of 10 to 250 mM, and preferably 25 mM to 200 mM, for example, 25 mM to 130 mM;
and/or the sodium chloride has a concentration of 30 to 250 mM, and preferably 50 mM to 170 mM, for example, 50 mM, 90mM, 130 mM, 145 mM or 170 mM;
and/or the methionine has a concentration of 5 to 50 mM, and preferably 5 mM, 10 mM or 50 mM;
and/or the mannitol has a concentration of 150 to 300 mM, for example, 165 mM, 220 mM or 280 mM.

4. The formulation according to claim 3, wherein the stabilizer has a concentration of 185 to 380 mM, and comprises at least mannitol and methionine, as well as trehalose or sucrose, wherein the mannitol has a concentration of 150 to 280 mM, the methionine has a concentration of 5 to 50 mM, and the trehalose or sucrose has a concentration of 25 to 130 mM;
and/or the recombinant human HAase has an enzyme activity of 150 units/ml to 4500000 units/ml.

5. The formulation according to claim 1, wherein the buffering agent is one or more of a histidine buffering agent, an acetic acid buffering agent, a phosphate buffering agent, a citric acid buffering agent, and a Tris buffering agent; for example, the buffering agent is a histidine buffering agent, an acetic acid buffering agent, a phosphate buffering agent, a citric acid buffering agent or a Tris buffering agent; and/or
the nonionic surfactant is one or more selected from polysorbate 20, polysorbate 80, and Poloxamer188,
wherein
the concentration of the acetic acid buffering agent is preferably 5 to 50 mM, for example, 5 mM, 10 mM or 50 mM;
the concentration of the phosphate buffering agent is preferably 5 to 50 mM, for example, 5 mM, 10 mM or 50 mM;
the concentration of the citric acid buffering agent is preferably 5 to 50 mM, for example, 5 mM, 10 mM or 50 mM;
the concentration of the Tris buffering agent is preferably 5 to 50 mM, for example, 5 mM, 10 mM or 50 mM; and
the concentration of the nonionic surfactant is preferably 0.02% (w/v).

6. The formulation according to any one of claims 1 to 5, wherein the stabilizer useful in the recombinant human HAase formulation has a composition selected from:
(1) 145 mM sodium chloride and 10 mM methionine;
(2) 130 mM sodium chloride, 53 mM trehalose and 10 mM methionine;
(3) 130 mM sodium chloride, 53 mM sucrose and 10 mM methionine;
(4) 26 mM trehalose, 280 mM mannitol and 10 mM methionine;
(5) 30 mM sodium chloride, 200 mM trehalose and 10 mM methionine;
(6) 180 mM sodium chloride, 25 mM trehalose and 10 mM methionine;
(7) 30 mM sodium chloride, 200 mM sucrose and 10 mM methionine;
(8) 180 mM sodium chloride, 25 mM sucrose and 10 mM methionine;
(9) 130 mM sodium chloride, 53 mM trehalose and 5 mM methionine;
(10) 130 mM sodium chloride, 53 mM trehalose and 50 mM methionine;
(11) 130 mM sodium chloride, 53 mM trehalose and 10 mM methionine;
(12) 22 mM mannitol, 53 mM trehalose and 10 mM methionine;
(13) 53 mM trehalose, 220 mM mannitol and 10 mM methionine;
(14) 53 mM trehalose, 220 mM mannitol and 5 mM methionine;
(15) 53 mM trehalose, 220 mM mannitol and 50 mM methionine;
(16) 150 mM mannitol, 170 mM sodium chloride and 10 mM methionine;
(17) 220 mM mannitol, 53 mM trehalose and 10 mM methionine;
(18) 150 mM mannitol, 50 mM sodium chloride, 53 mM trehalose and 10 mM methionine;
(19) 150 mM mannitol, 90 mM sodium chloride, 130 mM trehalose and 10 mM methionine;
(20) 150 mM mannitol, 25 mM trehalose and 10 mM methionine;
(21) 220 mM mannitol, 53 mM sucrose and 10 mM methionine;
(22) 150 mM mannitol, 50 mM sodium chloride, 53 mM sucrose and 10 mM methionine;
(23) 150 mM mannitol, 90 mM sodium chloride, 130 mM sucrose and 10mM methionine;
(24) 150 mM mannitol, 25 mM sucrose and 10 mM methionine; and
(25) 132 mM trehalose, 150 mM mannitol and 10 mM methionine.

7. The formulation according to claim 6, wherein the recombinant human HAase formulation has a composition selected from:
(1) 5000 units/ml recombinant human HAase, 5 mM phosphate buffering agent, 53 mM trehalose, 130 mM sodium chloride, 10 mM methionine and 0.02% (w/v) polysorbate 20, pH 7.0;
(2) 5000 units/ml recombinant human HAase, 10 mM phosphate buffering agent, 53 mM trehalose, 130 mM sodium chloride, 10 mM methionine and 0.02% (w/v) polysorbate 20, pH 7.0;
(3) 5000 units/ml recombinant human HAase, 50 mM phosphate buffering agent, 53 mM trehalose, 130 mM sodium chloride, 10 mM methionine and 0.02% (w/v) polysorbate 20, pH 7.0;
(4) 5000 units/ml recombinant human HAase, 5 mM histidine buffering agent, 53 mM trehalose, 130 mM sodium chloride, 10 mM methionine and 0.02% (w/v) polysorbate 20, pH 6.0;
(5) 5000 units/ml recombinant human HAase, 50 mM histidine buffering agent, 53 mM trehalose, 130 mM sodium chloride, 10 mM methionine and 0.02% (w/v) polysorbate 20, pH 6.0;
(6) 5000 units/ml recombinant human HAase, 5 mM acetic acid buffering agent, 53 mM trehalose, 130 mM sodium chloride, 10 mM methionine and 0.02% (w/v) polysorbate 20, pH 5.0;
(7) 5000 units/ml recombinant human HAase, 50 mM acetic acid buffering agent, 53 mM trehalose, 130 mM sodium chloride, 10 mM methionine and 0.02% (w/v) polysorbate 20, pH 5.0;
(8) 5000 units/ml recombinant human HAase, 5 mM citric acid buffering agent, 53 mM trehalose, 130 mM sodium chloride, 10 mM methionine and 0.02% (w/v) polysorbate 20, pH 6.5;
(9) 5000 units/ml recombinant human HAase, 50 mM citric acid buffering agent, 53 mM trehalose, 130 mM sodium chloride, 10 mM methionine and 0.02% (w/v) polysorbate 20, pH 6.5;
(10) 5000 units/ml recombinant human HAase, 5 mM Tris buffering agent, 53 mM trehalose, 130 mM sodium chloride, 10 mM methionine and 0.02% (w/v) polysorbate 20, pH 8.0;
(11) 5000 units/ml recombinant human HAase, 50 mM Tris buffering agent, 53 mM trehalose, 130 mM sodium chloride, 10 mM methionine and 0.02% (w/v) polysorbate 20, pH 8.0;
(12) 5000 units/ml recombinant human HAase, 10 mM phosphate buffering agent, 53 mM trehalose, 130 mM sodium chloride, 5 mM methionine and 0.02% (w/v) polysorbate 20, pH 7.0;
(13) 5000 units/ml recombinant human HAase, 10 mM phosphate buffering agent, 53 mM trehalose, 130 mM sodium chloride, 50 mM methionine and 0.02% (w/v) polysorbate 20, pH 7.0;
(14) 5000 units/ml recombinant human HAase, 10 mM phosphate buffering agent, 200 mM trehalose, 30 mM sodium chloride, 10 mM methionine and 0.02% (w/v) polysorbate 20, pH 7.0;
(15) 5000 units/ml recombinant human HAase, 10 mM phosphate buffering agent, 53 mM trehalose, 130 mM sodium chloride, 10 mM methionine and 0.02% (w/v) Poloxamer188, pH 7.0;
(16) 5000 units/ml recombinant human HAase, 10 mM phosphate buffering agent, 25 mM trehalose, 180 mM sodium chloride, 10 mM methionine and 0.02% (w/v) polysorbate 20, pH 7.0;
(17) 5000 units/ml recombinant human HAase, 10 mM phosphate buffering agent, 200 mM sucrose, 30 mM sodium chloride, 10 mM methionine and 0.02% (w/v) polysorbate 20, pH 7.0;
(18) 5000 units/ml recombinant human HAase, 10 mM phosphate buffering agent, 53 mM sucrose, 130 mM sodium chloride, 10 mM methionine and 0.02% (w/v) polysorbate 20, pH 7.0;
(19) 5000 units/ml recombinant human HAase, 10 mM phosphate buffering agent, 25 mM sucrose, 180 mM sodium chloride, 10 mM methionine and 0.02% (w/v) polysorbate 20, pH 7.0;
(20) 5000 units/ml recombinant human HAase, 10 mM phosphate buffering agent, 53 mM trehalose, 130 mM sodium chloride, 10 mM methionine and 0.02% (w/v) polysorbate 80, pH 7.0;
(21) 5000 units/ml recombinant human HAase, 10 mM phosphate buffering agent, 53 mM trehalose, 130 mM sodium chloride, 10 mM methionine and 0.01% (w/v) polysorbate 80, pH 7.0;
(22) 5000 units/ml recombinant human HAase, 10 mM phosphate buffering agent, 53 mM trehalose, 130 mM sodium chloride, 10 mM methionine and 0.10% (w/v) polysorbate 80, pH 7.0;
(23) 5000 units/ml recombinant human HAase, 10 mM phosphate buffering agent, 53 mM trehalose, 130 mM sodium chloride, 10 mM methionine and 0.01% (w/v) polysorbate 20, pH 7.0;
(24) 5000 units/ml recombinant human HAase, 10 mM phosphate buffering agent, 53 mM trehalose, 130 mM sodium chloride, 10 mM methionine and 0.10% (w/v) polysorbate 20, pH 7.0;
(25) 5000 units/ml recombinant human HAase, 10 mM phosphate buffering agent, 53 mM trehalose, 130 mM sodium chloride, 10 mM methionine and 0.01% (w/v) Poloxamer188, pH 7.0;
(26) 5000 units/ml recombinant human HAase, 10 mM phosphate buffering agent, 53 mM trehalose, 130 mM sodium chloride, 10 mM methionine and 0.10% (w/v) Poloxamer188, pH 7.0;
(27) 500 units/ml recombinant human HAase, 10 mM phosphate buffering agent, 53 mM trehalose, 130 mM sodium chloride, 10 mM methionine and 0.02 (w/v) polysorbate 20, pH 7.0;
(28) 45 units/ml recombinant human HAase, 10 mM phosphate buffering agent, 53 mM trehalose, 130 mM sodium chloride, 10 mM methionine and 0.02 (w/v) polysorbate 20, pH 7.0;
(29) 150 units/ml recombinant human HAase, 10 mM phosphate buffering agent, 53 mM trehalose, 130 mM sodium chloride, 10 mM methionine and 0.02 (w/v) polysorbate 20, pH 7.0;
(30) 1000 units/ml recombinant human HAase, 10 mM phosphate buffering agent, 53 mM trehalose, 130 mM sodium chloride, 10 mM methionine and 0.02 (w/v) polysorbate 20, pH 7.0;
(31) 50000 units/ml recombinant human HAase, 10 mM phosphate buffering agent, 53 mM trehalose, 130 mM sodium chloride, 10 mM methionine and 0.02 (w/v) polysorbate 20, pH 7.0;
(32) 300000 units/ml recombinant human HAase, 10 mM phosphate buffering agent, 53 mM trehalose, 130 mM sodium chloride, 10 mM methionine and 0.02 (w/v) polysorbate 20, pH 7.0;
(33) 1500000 units/ml recombinant human HAase, 10 mM phosphate buffering agent, 53 mM trehalose, 130 mM sodium chloride, 10 mM methionine and 0.02 (w/v) polysorbate 20, pH 7.0;
(34) 3000000 units/ml recombinant human HAase, 10 mM phosphate buffering agent, 53 mM trehalose, 130 mM sodium chloride, 10 mM methionine and 0.02 (w/v) polysorbate 20, pH 7.0;
(35) 4500000 units/ml recombinant human HAase, 10 mM phosphate buffering agent, 53 mM trehalose, 130 mM sodium chloride, 10 mM methionine and 0.02 (w/v) polysorbate 20, pH 7.0;
(36) 4500000 units/ml recombinant human HAase, 10 mM phosphate buffering agent, 145 mM sodium chloride, 10 mM methionine and 0.02 (w/v) polysorbate 20, pH 7.0;
(37) 1500000 units/ml recombinant human HAase, 10 mM phosphate buffering agent, 145 mM sodium chloride, 10 mM methionine and 0.02 (w/v) polysorbate 20, pH 7.0;
(38) 300000 units/ml recombinant human HAase, 10 mM phosphate buffering agent, 145 mM sodium chloride, 10 mM methionine and 0.02 (w/v) polysorbate 20, pH 7.0;
(39) 5000 units/ml recombinant human HAase, 10 mM phosphate buffering agent, 145 mM sodium chloride, 10 mM methionine and 0.02 (w/v) polysorbate 20, pH 7.0;
(40) 150 units/ml recombinant human HAase, 10 mM phosphate buffering agent, 145 mM sodium chloride, 10 mM methionine and 0.02 (w/v) polysorbate 20, pH 7.0;
(41) 45 units/ml recombinant human HAase, 10 mM phosphate buffering agent, 145 mM sodium chloride, 10 mM methionine and 0.02 (w/v) polysorbate 20, pH 7.0;
(42) 45 units/ml recombinant human HAase, 10 mM phosphate buffering agent, 22 mM mannitol, 53 mM trehalose, 10 mM methionine and 0.02% polysorbate 20, pH 7.0;
(43) 500 units/ml recombinant human HAase, 10 mM phosphate buffering agent, 22 mM mannitol, 53 mM trehalose, 10 mM methionine and 0.02% polysorbate 20, pH 7.0;
(44) 5000 units/ml recombinant human HAase, 10 mM phosphate buffering agent, 22 mM mannitol, 53 mM trehalose, 10 mM methionine and 0.02% polysorbate 20, pH 7.0;
(45) 50000 units/ml recombinant human HAase, 10 mM phosphate buffering agent, 22 mM mannitol, 53 mM trehalose, 10 mM methionine and 0.02% polysorbate 20, pH 7.0;
(46) 300000 units/ml recombinant human HAase, 10 mM phosphate buffering agent, 22 mM mannitol, 53 mM trehalose, 10 mM methionine and 0.02% polysorbate 20, pH 7.0;
(47) 4500000 units/ml recombinant human HAase, 10 mM phosphate buffering agent, 22 mM mannitol, 53 mM trehalose, 10 mM methionine and 0.02% polysorbate 20, pH 7.0;
(48) 5000 units/ml recombinant human HAase, 5 mM phosphate buffering agent, 53 mM trehalose, 220 mM mannitol, 10 mM methionine and 0.02% polysorbate 20, pH 7.0;
(49) 5000 units/ml recombinant human HAase, 10 mM phosphate buffering agent, 53 mM trehalose, 220 mM mannitol, 10 mM methionine and 0.02% polysorbate 20, pH 7.0;
(50) 5000 units/ml recombinant human HAase, 50 mM phosphate buffering agent, 53 mM trehalose, 220 mM mannitol, 10 mM methionine and 0.02% polysorbate 20, pH 7.0;
(51) 5000 units/ml recombinant human HAase, 5 mM phosphate buffering agent, 53 mM trehalose, 220 mM mannitol, 10 mM methionine and 0.02% polysorbate 20, pH 6.0;
(52) 5000 units/ml recombinant human HAase, 50 mM phosphate buffering agent, 53 mM trehalose, 220 mM mannitol, 10 mM methionine and 0.02% polysorbate 20, pH 6.0;
(53) 5000 units/ml recombinant human HAase, 50 mM acetic acid buffering agent, 53 mM trehalose, 220 mM mannitol, 10 mM methionine and 0.02% polysorbate 20, pH 5.0;
(54) 5000 units/ml recombinant human HAase, 5 mM acetic acid buffering agent, 53 mM trehalose, 220 mM mannitol, 10 mM methionine and 0.02% polysorbate 20, pH 5.0;
(55) 5000 units/ml recombinant human HAase, 50 mM citric acid buffering agent, 53 mM trehalose, 220 mM mannitol, 10 mM methionine and 0.02% polysorbate 20, pH 6.5;
(56) 5000 units/ml recombinant human HAase, 5 mM citric acid buffering agent, 53 mM trehalose, 220 mM mannitol, 10 mM methionine and 0.02% polysorbate 20, pH 6.5;
(57) 5000 units/ml recombinant human HAase, 50 mM Tris buffering agent, 53 mM trehalose, 220 mM mannitol, 10 mM methionine and 0.02% polysorbate 20, pH 8.0;
(58) 5000 units/ml recombinant human HAase, 5 mM Tris buffering agent, 53 mM trehalose, 220 mM mannitol, 10 mM methionine and 0.02% polysorbate 20, pH 8.0;
(59) 5000 units/ml recombinant human HAase, 10 mM phosphate buffering agent, 53 mM trehalose, 220 mM mannitol, 50 mM methionine and 0.02% polysorbate 20, pH 7.0;
(60) 5000 units/ml recombinant human HAase, 10 mM phosphate buffering agent, 53 mM trehalose, 220 mM mannitol, 5 mM methionine and 0.02% polysorbate 20, pH 7.0;
(61) 5000 units/ml recombinant human HAase, 10 mM phosphate buffering agent, 150 mM mannitol, 170 mM sodium chloride, 10 mM methionine and 0.02% polysorbate 20, pH 7.0;
(62) 5000 units/ml recombinant human HAase, 10 mM phosphate buffering agent, 220 mM mannitol, 53 mM trehalose, 10 mM methionine and 0.02% polysorbate 20, pH 7.0;
(63) 5000 units/ml recombinant human HAase, 10 mM phosphate buffering agent, 150 mM mannitol, 50 mM sodium chloride, 53 mM trehalose, 10 mM methionine and 0.02% polysorbate 20, pH 7.0;
(64) 5000 units/ml recombinant human HAase, 10 mM phosphate buffering agent, 150 mM mannitol, 90 mM sodium chloride, 130 mM trehalose, 10 mM methionine and 0.02% polysorbate 20, pH 7.0;
(65) 5000 units/ml recombinant human HAase, 10 mM phosphate buffering agent, 150 mM mannitol, 25 mM trehalose, 10 mM methionine and 0.02% polysorbate 20, pH 7.0;
(66) 5000 units/ml recombinant human HAase, 10 mM phosphate buffering agent, 220 mM mannitol, 53 mM sucrose, 10 mM methionine and 0.02% polysorbate 20, pH 7.0;
(67) 5000 units/ml recombinant human HAase, 10 mM phosphate buffering agent, 150 mM mannitol, 50 mM sodium chloride, 53 mM sucrose, 10 mM methionine and 0.02% polysorbate 20, pH 7.0;
(68) 5000 units/ml recombinant human HAase, 10 mM phosphate buffering agent, 150 mM mannitol, 90 mM sodium chloride, 130 mM sucrose, 10mM methionine and 0.02% polysorbate 20, pH 7.0;
(69) 5000 units/ml recombinant human HAase, 10 mM phosphate buffering agent, 150 mM mannitol, 25 mM sucrose, 10 mM methionine and 0.02% polysorbate 20, pH 7.0;
(70) 5000 units/ml recombinant human HAase, 10 mM phosphate buffering agent, 132 mM trehalose, 150 mM mannitol, 10 mM methionine and 0.02% polysorbate 20, pH 7.0;
(71) 5000 units/ml recombinant human HAase, 10 mM phosphate buffering agent, 26 mM trehalose, 280 mM mannitol, 10 mM methionine and 0.02% polysorbate 20, pH 7.0;
(72) 5000 units/ml recombinant human HAase, 10 mM phosphate buffering agent, 53 mM trehalose, 220 mM mannitol, 10 mM methionine and 0.01% polysorbate 20, pH 7.0;
(73) 5000 units/ml recombinant human HAase, 10 mM phosphate buffering agent, 53 mM trehalose, 220 mM mannitol, 10 mM methionine and 0.10% polysorbate 20, pH 7.0;
(74) 5000 units/ml recombinant human HAase, 10 mM phosphate buffering agent, 53 mM trehalose, 220 mM mannitol, 10 mM methionine and 0.01% polysorbate 80, pH 7.0;
(75) 5000 units/ml recombinant human HAase, 10 mM phosphate buffering agent, 53 mM trehalose, 220 mM mannitol, 10 mM methionine and 0.10% polysorbate 80, pH 7.0;
(76) 5000 units/ml recombinant human HAase, 10 mM phosphate buffering agent, 53 mM trehalose, 220 mM mannitol, 10 mM methionine and 0.02% polysorbate 80, pH 7.0;
(77) 5000 units/ml recombinant human HAase, 10 mM phosphate buffering agent, 53 mM trehalose, 220 mM mannitol, 10 mM methionine and 0.10% Poloxamer 188, pH 7.0;
(78) 5000 units/ml recombinant human HAase, 10 mM phosphate buffering agent, 53 mM trehalose, 220 mM mannitol, 10 mM methionine and 0.01% Poloxamer 188, pH 7.0;
(79) 5000 units/ml recombinant human HAase, 10 mM phosphate buffering agent, 53 mM trehalose, 220 mM mannitol, 10 mM methionine and 0.02% Poloxamer 188, pH 7.0;
(80) 150 units/ml recombinant human HAase, 10 mM phosphate buffering agent, 53 mM trehalose, 220 mM mannitol, 10 mM methionine and 0.02% polysorbate 20, pH 7.0;
(81) 500 units/ml recombinant human HAase, 10 mM phosphate buffering agent, 53 mM trehalose, 220 mM mannitol, 10 mM methionine and 0.02% polysorbate 20, pH 7.0;
(82) 1000 units/ml recombinant human HAase, 10 mM phosphate buffering agent, 53 mM trehalose, 220 mM mannitol, 10 mM methionine and 0.02% polysorbate 20, pH 7.0;
(83) 300000 units/ml recombinant human HAase, 10 mM phosphate buffering agent, 53 mM trehalose, 220 mM mannitol, 10 mM methionine and 0.02% polysorbate 20, pH 7.0;
(84) 50000 units/ml recombinant human HAase, 10 mM phosphate buffering agent, 53 mM trehalose, 220 mM mannitol, 10 mM methionine and 0.02% polysorbate 20, pH 7.0;
(85) 1500000 units/ml recombinant human HAase, 10 mM phosphate buffering agent, 53 mM trehalose, 220 mM mannitol, 10 mM methionine and 0.02% polysorbate 20, pH 7.0;
(86) 3000000 units/ml recombinant human HAase, 10 mM phosphate buffering agent, 53 mM trehalose, 220 mM mannitol, 10 mM methionine and 0.02% polysorbate 20, pH 7.0; and
(87) 4500000 units/ml recombinant human HAase, 10 mM phosphate buffering agent, 53 mM trehalose, 220 mM mannitol, 10 mM methionine and 0.02% polysorbate 20, pH 7.0.

8. Use of the formulation according to any one of claims 1 to 7 in the formulation of a subcutaneous injection promoting agent.

9. The use according to claim 8, wherein the subcutaneous injection promoting agent comprises at least the recombinant human HAase formulation.

10. The use according to claim 9, wherein the drug for subcutaneous injection comprises both or either of a drug carrier and/or a drug.

11. The use according to claim 10, wherein the drug carrier comprises a glucose solution, an isotonic electrolyte solution, an alkaline solution, a hypertonic solution, dextran, a syrup substitute or a blood product.

12. The use according to claim 10, wherein the drug carrier is deemed as an agent having therapeutic effect in clinical use in some cases.

13. The use according to claim 10, wherein the drug comprises chemotherapeutic agents, analgesics, anti-inflammatory agents, antimicrobial agents, anti-amoebic agents, trichomonacidal agents, anti-parkinsonism agent, anti-dysentery agents, antispasmodic agents, antidepressants, antirheumatic agents, antifungal agents, antihypertensive agents, antipyretics, antiparasitics, antihistamines, α-adrenergic agonists, α-blocking agents, anesthetics, bronchodilators, biocidal agents, bactericides, bacteriostatic agents, β -adrenergic blocking agents, calcium channel blocking agents, cardiovascular agents, contraceptives, decongestants, diuretics, sedatives, diagnostic agents, electrolyte agents, hypnotic agents, hormones, hyperglycemic agents, muscle relaxants, muscle contracting agents, ophthalmic agents, parasympathomimetics, psychostimulants, tranquilizers, sympathomimetics, tranquilizer, urinary agents, vaginal agents, antiviral agents, vitamin agents, non-steroidal anti-inflammatory agents, angiotensin converting enzyme inhibitors, polypeptides, proteins, nucleic acids, organic molecules, sleep promoters, insulin, cytokines, antibodies and monoclonal antibodies.

14. A drug combination, comprising the recombinant human HAase formulation according to any one of claims 1 to 7 and a drug for subcutaneous injection.

15. The drug combination according to claim 14, further comprising a drug carrier for the drug for subcutaneous injection, wherein
the drug for subcutaneous injection is packaged into
(1) a separate package;
(2) a drug combination with a drug carrier to be promoted for subcutaneous injection; and
(3) a drug combination with a drug to be promoted for subcutaneous injection.

16. The drug combination according to claim 14, wherein the drug for subcutaneous injection is a recombinant protein, a fusion protein, human albumin, immunoglobulin, a targeting antibody drug, a polypeptide, nanoparticles, viruses, cells or chemicals,
wherein the polypeptide is preferably insulin, glucagon-like peptide-1 (GLP-1) analog, leuprorelin, vasopressin, and bacitracin; or
the drug for subcutaneous injection is preferably a drug having anti-tumor effect, wherein
the drug having anti-tumor effect is preferably one or more of oncolytic viruses, immune cells, CAR-T cells, TCR-T cells, anti-tumor drugs, antibody drugs targeting tumors, ADC antibody drugs, immune checkpoint inhibitors, nanoparticle drugs and corticosteroids, wherein
the antibody drug targeting tumor is preferably one or two of trastuzumab, rituximab, pertuzumab, daratumumab and Isatuximab, for example, rituximab, trastuzumab and pertuzumab, and the concentration of the antibody is preferably 60 to 120 mg/ml, wherein
when pertuzumab and trastuzumab are used in combination, the dose of pertuzumab is 300 to 1500 mg, and preferably 600 mg or 1200 mg; and the dose of trastuzumab is 200 to 800 mg, and preferably 600 mg; and
when rituximab is used alone, the dose of rituximab is 700 to 1600 mg, and preferably 1400 mg or 1600 mg.

17. The drug combination according to any one of claims 14 to 16, which is a combination formulation or a kit consisting of separate packages of non-combination formulations.

18. The drug combination according to claim 17, wherein the steps of administering the combination formulation to a subject in need of treatment comprise:
formulating the combination formulation, optionally added with or without a desired drug, into a solution intended to be injected, and then intradermally or subcutaneously administrating the solution to be injected to the subject in need of treatment.

19. The drug combination according to claim 17, wherein the steps of administering the combination formulation to a subject in need of treatment comprise:
dissolving the combination formulation in a suitable drug carrier, to formulate a solution intended to be injected, and then intradermally or subcutaneously administrating the solution to be injected to the subject in need of treatment.

20. The drug combination according to claim 17, wherein the steps of administering the kit to a subject in need of treatment comprise:
(a) formulating the subcutaneous injection promoting agent in a separate formulation package and the drug carrier in a separate formulation package, optionally mixed with/without a suitable drug, into a solution intended to be injected, and then intradermally or subcutaneously administrating the solution to be injected to the subject in need of treatment; or
(b) formulating the drug carrier in the kit, optionally mixed with/without a suitable drug, into a solution to be injected, intradermally or subcutaneously administrating the subcutaneous injection promoting agent in the kit to the subject in need of treatment, and then administrating the solution to be injected to the subject in need of treatment.

21. The drug combination according to claim 17, wherein the steps of administering the kit to a subject in need of treatment comprise:
(a) mixing the subcutaneous injection promoting agent in a separate formulation package, the drug in a separate formulation package and a suitable drug carrier, formulating a solution intended to be injected, and then intradermally or subcutaneously administrating the solution to be injected to the subject in need of treatment; or
(b) mixing the drug in the kit with a suitable drug carrier, formulating a solution to be injected, intradermally or subcutaneously administrating the subcutaneous injection promoting agent in the kit to the subject in need of treatment, and then administrating the solution to be injected to the subject in need of treatment.

22. The drug combination according to claim 21, wherein the step of administering the subcutaneous injection promoting agent to the subject in need of treatment comprises:
(a) formulating the subcutaneous injection promoting agent and a suitable drug carrier, optionally mixed with/without a suitable drug, into a solution intended to be injected, and then intradermally or subcutaneously administrating the solution to be injected to the subject in need of treatment; or
(b) formulating a suitable drug carrier, optionally mixed with/without a suitable drug, into a solution to be injected, intradermally or subcutaneously administrating the subcutaneous injection promoting agent to the subject in need of treatment, and then administrating the solution to be injected to the subject in need of treatment.

23. A method for treating a disease, comprising administering the recombinant human HAase formulation according to any one of claims 1 to 7 or the drug combination according to any one of claims 14 to 22 to a subject in need of treatment, wherein
the administration comprises preferably administering separately or in mixture.

24. The method according to claim 23, wherein the separate administration comprises the following steps:
(a) intradermally or subcutaneously administrating the recombinant human HAase formulation in the kit to the subject in need of treatment; and
(b) then administrating the drug for subcutaneous injection in the kit to the subject,
wherein Steps (a) and (b) are performed separately, simultaneously or alternatively; and preferably,
when Steps (a) and (b) are performed separately, the time interval between Steps (a) and (b) is 0 to 24 hrs, and
preferably, the time interval is 0 min, at most 1 min, at most 2 min, at most 3 min, at most 4 min, at most 5 min, at most 6 min, at most 7 min, at most 8 min, at most 9 min, at most 10 min, at most 15 min, at most 20 min, at most 25 min, at most 30 min, at most 1 hr, at most 2 hrs, at most 3 hrs, at most 6 hrs, at most 12 hrs or at most 24 hrs.

25. The method according to claim 24, wherein in the case of sequential subcutaneous administration, the time interval between Steps (a) and (b) is less than 10 min, and preferably 0 min, 1 min, 2 min, 3 min, 4 min, 5 min, 6 min, 7 min, 8 min or 9 min.

26. The method according to any one of claims 23 to 25, wherein in the case of separate administration, simultaneous administration or sequential administration is enabled by means of a three-way valve; and
upon separate administration, the administration rate is controlled by an injection pump or by gravity,
wherein the recombinant human HAase formulation is dosed at a rate of 0.1 to 2 ml/min; and
the drug for subcutaneous injection is injected at a rate of 5 ml/hr, 10 ml/hr, 30 ml/hr, 60 ml/hr, 120 ml/hr, 240 ml/hr or 300 ml/hr.

27. The method according to claim 23, wherein the administration in mixture comprises mixing the recombinant human HAase formulation and the drug for subcutaneous injection in the kit and intradermally or subcutaneously administrating the mixture to the subject, wherein
the drug for subcutaneous injection intended to be mixed is preferably in the form of a liquid or a dry powder.

28. The method according to any one of claims 23 to 27, wherein the subject in need of treatment is a subject having local edema or hematoma after surgery or trauma, receiving local anesthetics, receiving infusion or receiving the injection of the contrast agent barium sulfate; and
preferably the subject in need of treatment has accumulated drug solution, exudate, or blood locally in the eyes, vitreous opacities, chemical burn of conjunctiva accompanied by inflammation, traumatic orbital hemorrhage, or traumatic retinal edema.

## Amended claims

### Amended claims under Art. 19.1 PCT

1. A recombinant human hyaluronidase (HAase) formulation, comprising a recombinant human HAase, a buffering agent, a stabilizer and a nonionic surfactant, wherein
the recombinant human HAase has an enzyme activity of 45 units/ml to 4500000 units/ml;
the buffering agent has a concentration of 5 to 50 mM;
the stabilizer has a concentration of 1 to 500 mM, and the stabilizer comprises methionine, optionally further comprises one or more selected from trehalose, sucrose, mannitol, and sodium chloride;
the nonionic surfactant has a concentration of 0.01% to 0.1% (w/v); and
the formulation has a pH of 5.0 to 8.0.

2. The formulation according to claim 1, wherein the recombinant human HAase has an enzyme activity of 45 units/ml to 3000000 units/ml, preferably 45 units/ml to 1500000 units/ml, and further preferably 45 units/ml to 300000 units/ml;
and/or the recombinant human HAase comprises an amino acid sequence as shown in SEQ ID NO. 1;
and preferably the recombinant human HAase has an amino acid sequence as shown in SEQ ID NO. 2;
and/or the stabilizer has a concentration of 100 to 400 mM, and preferably 150 to 350 mM, for example, 155 mM, 188 mM, 193 mM, 215 mM, 233 mM, 240 mM, 263 mM, 283 mM, 292 mM, 316 mM or 330 mM.

3. The formulation according to claim 1, wherein
the trehalose or sucrose has a concentration of 10 to 250 mM, and preferably 25 mM to 200 mM, for example, 25 mM to 130 mM;
and/or the sodium chloride has a concentration of 30 to 250 mM, and preferably 50 mM to 170 mM, for example, 50 mM, 90mM, 130 mM, 145 mM or 170 mM;
and/or the methionine has a concentration of 5 to 50 mM, and preferably 5 mM, 10 mM or 50 mM;
and/or the mannitol has a concentration of 150 to 300 mM, for example, 165 mM, 220 mM or 280 mM.

4. The formulation according to claim 3, wherein the stabilizer has a concentration of 185 to 380 mM, and comprises mannitol, methionine, as well as trehalose or sucrose, wherein the mannitol has a concentration of 150 to 280 mM, the methionine has a concentration of 5 to 50 mM, and the trehalose or sucrose has a concentration of 25 to 130 mM;
and/or the recombinant human HAase has an enzyme activity of 150 units/ml to 4500000 units/ml.

5. The formulation according to claim 1, wherein the buffering agent is one or more of a histidine buffering agent, an acetic acid buffering agent, a phosphate buffering agent, a citric acid buffering agent, and a Tris buffering agent; for example, the buffering agent is a histidine buffering agent, an acetic acid buffering agent, a phosphate buffering agent, a citric acid buffering agent or a Tris buffering agent; and/or
the nonionic surfactant is one or more selected from polysorbate 20, polysorbate 80, and Poloxamer188, wherein
the concentration of the acetic acid buffering agent is preferably 5 to 50 mM, for example, 5 mM, 10 mM or 50 mM;
the concentration of the phosphate buffering agent is preferably 5 to 50 mM, for example, 5 mM, 10 mM or 50 mM;
the concentration of the citric acid buffering agent is preferably 5 to 50 mM, for example, 5 mM, 10 mM or 50 mM;
the concentration of the Tris buffering agent is preferably 5 to 50 mM, for example, 5 mM, 10 mM or 50 mM; and
the concentration of the nonionic surfactant is preferably 0.02% (w/v).

6. The formulation according to any one of claims 1 to 5, wherein the stabilizer useful in the recombinant human HAase formulation has a composition selected from:
(1) 145 mM sodium chloride and 10 mM methionine;
(2) 130 mM sodium chloride, 53 mM trehalose and 10 mM methionine;
(3) 130 mM sodium chloride, 53 mM sucrose and 10 mM methionine;
(4) 26 mM trehalose, 280 mM mannitol and 10 mM methionine;
(5) 30 mM sodium chloride, 200 mM trehalose and 10 mM methionine;
(6) 180 mM sodium chloride, 25 mM trehalose and 10 mM methionine;
(7) 30 mM sodium chloride, 200 mM sucrose and 10 mM methionine;
(8) 180 mM sodium chloride, 25 mM sucrose and 10 mM methionine;
(9) 130 mM sodium chloride, 53 mM trehalose and 5 mM methionine;
(10) 130 mM sodium chloride, 53 mM trehalose and 50 mM methionine;
(11) 130 mM sodium chloride, 53 mM trehalose and 10 mM methionine;
(12) 22 mM mannitol, 53 mM trehalose and 10 mM methionine;
(13) 53 mM trehalose, 220 mM mannitol and 10 mM methionine;
(14) 53 mM trehalose, 220 mM mannitol and 5 mM methionine;
(15) 53 mM trehalose, 220 mM mannitol and 50 mM methionine;
(16) 150 mM mannitol, 170 mM sodium chloride and 10 mM methionine;
(17) 220 mM mannitol, 53 mM trehalose and 10 mM methionine;
(18) 150 mM mannitol, 50 mM sodium chloride, 53 mM trehalose and 10 mM methionine;
(19) 150 mM mannitol, 90 mM sodium chloride, 130 mM trehalose and 10 mM methionine;
(20) 150 mM mannitol, 25 mM trehalose and 10 mM methionine;
(21) 220 mM mannitol, 53 mM sucrose and 10 mM methionine;
(22) 150 mM mannitol, 50 mM sodium chloride, 53 mM sucrose and 10 mM methionine;
(23) 150 mM mannitol, 90 mM sodium chloride, 130 mM sucrose and 10mM methionine;
(24) 150 mM mannitol, 25 mM sucrose and 10 mM methionine; and
(25) 132 mM trehalose, 150 mM mannitol and 10 mM methionine.

7. The formulation according to claim 6, wherein the recombinant human HAase formulation has a composition selected from:
(1) 5000 units/ml recombinant human HAase, 5 mM phosphate buffering agent, 53 mM trehalose, 130 mM sodium chloride, 10 mM methionine and 0.02% (w/v) polysorbate 20, pH 7.0;
(2) 5000 units/ml recombinant human HAase, 10 mM phosphate buffering agent, 53 mM trehalose, 130 mM sodium chloride, 10 mM methionine and 0.02% (w/v) polysorbate 20, pH 7.0;
(3) 5000 units/ml recombinant human HAase, 50 mM phosphate buffering agent, 53 mM trehalose, 130 mM sodium chloride, 10 mM methionine and 0.02% (w/v) polysorbate 20, pH 7.0;
(4) 5000 units/ml recombinant human HAase, 5 mM histidine buffering agent, 53 mM trehalose, 130 mM sodium chloride, 10 mM methionine and 0.02% (w/v) polysorbate 20, pH 6.0;
(5) 5000 units/ml recombinant human HAase, 50 mM histidine buffering agent, 53 mM trehalose, 130 mM sodium chloride, 10 mM methionine and 0.02% (w/v) polysorbate 20, pH 6.0;
(6) 5000 units/ml recombinant human HAase, 5 mM acetic acid buffering agent, 53 mM trehalose, 130 mM sodium chloride, 10 mM methionine and 0.02% (w/v) polysorbate 20, pH 5.0;
(7) 5000 units/ml recombinant human HAase, 50 mM acetic acid buffering agent, 53 mM trehalose, 130 mM sodium chloride, 10 mM methionine and 0.02% (w/v) polysorbate 20, pH 5.0;
(8) 5000 units/ml recombinant human HAase, 5 mM citric acid buffering agent, 53 mM trehalose, 130 mM sodium chloride, 10 mM methionine and 0.02% (w/v) polysorbate 20, pH 6.5;
(9) 5000 units/ml recombinant human HAase, 50 mM citric acid buffering agent, 53 mM trehalose, 130 mM sodium chloride, 10 mM methionine and 0.02% (w/v) polysorbate 20, pH 6.5;
(10) 5000 units/ml recombinant human HAase, 5 mM Tris buffering agent, 53 mM trehalose, 130 mM sodium chloride, 10 mM methionine and 0.02% (w/v) polysorbate 20, pH 8.0;
(11) 5000 units/ml recombinant human HAase, 50 mM Tris buffering agent, 53 mM trehalose, 130 mM sodium chloride, 10 mM methionine and 0.02% (w/v) polysorbate 20, pH 8.0;
(12) 5000 units/ml recombinant human HAase, 10 mM phosphate buffering agent, 53 mM trehalose, 130 mM sodium chloride, 5 mM methionine and 0.02% (w/v) polysorbate 20, pH 7.0;
(13) 5000 units/ml recombinant human HAase, 10 mM phosphate buffering agent, 53 mM trehalose, 130 mM sodium chloride, 50 mM methionine and 0.02% (w/v) polysorbate 20, pH 7.0;
(14) 5000 units/ml recombinant human HAase, 10 mM phosphate buffering agent, 200 mM trehalose, 30 mM sodium chloride, 10 mM methionine and 0.02% (w/v) polysorbate 20, pH 7.0;
(15) 5000 units/ml recombinant human HAase, 10 mM phosphate buffering agent, 53 mM trehalose, 130 mM sodium chloride, 10 mM methionine and 0.02% (w/v) Poloxamer188, pH 7.0;
(16) 5000 units/ml recombinant human HAase, 10 mM phosphate buffering agent, 25 mM trehalose, 180 mM sodium chloride, 10 mM methionine and 0.02% (w/v) polysorbate 20, pH 7.0;
(17) 5000 units/ml recombinant human HAase, 10 mM phosphate buffering agent, 200 mM sucrose, 30 mM sodium chloride, 10 mM methionine and 0.02% (w/v) polysorbate 20, pH 7.0;
(18) 5000 units/ml recombinant human HAase, 10 mM phosphate buffering agent, 53 mM sucrose, 130 mM sodium chloride, 10 mM methionine and 0.02% (w/v) polysorbate 20, pH 7.0;
(19) 5000 units/ml recombinant human HAase, 10 mM phosphate buffering agent, 25 mM sucrose, 180 mM sodium chloride, 10 mM methionine and 0.02% (w/v) polysorbate 20, pH 7.0;
(20) 5000 units/ml recombinant human HAase, 10 mM phosphate buffering agent, 53 mM trehalose, 130 mM sodium chloride, 10 mM methionine and 0.02% (w/v) polysorbate 80, pH 7.0;
(21) 5000 units/ml recombinant human HAase, 10 mM phosphate buffering agent, 53 mM trehalose, 130 mM sodium chloride, 10 mM methionine and 0.01% (w/v) polysorbate 80, pH 7.0;
(22) 5000 units/ml recombinant human HAase, 10 mM phosphate buffering agent, 53 mM trehalose, 130 mM sodium chloride, 10 mM methionine and 0.10% (w/v) polysorbate 80, pH 7.0;
(23) 5000 units/ml recombinant human HAase, 10 mM phosphate buffering agent, 53 mM trehalose, 130 mM sodium chloride, 10 mM methionine and 0.01% (w/v) polysorbate 20, pH 7.0;
(24) 5000 units/ml recombinant human HAase, 10 mM phosphate buffering agent, 53 mM trehalose, 130 mM sodium chloride, 10 mM methionine and 0.10% (w/v) polysorbate 20, pH 7.0;
(25) 5000 units/ml recombinant human HAase, 10 mM phosphate buffering agent, 53 mM trehalose, 130 mM sodium chloride, 10 mM methionine and 0.01% (w/v) Poloxamer188, pH 7.0;
(26) 5000 units/ml recombinant human HAase, 10 mM phosphate buffering agent, 53 mM trehalose, 130 mM sodium chloride, 10 mM methionine and 0.10% (w/v) Poloxamer188, pH 7.0;
(27) 500 units/ml recombinant human HAase, 10 mM phosphate buffering agent, 53 mM trehalose, 130 mM sodium chloride, 10 mM methionine and 0.02 (w/v) polysorbate 20, pH 7.0;
(28) 45 units/ml recombinant human HAase, 10 mM phosphate buffering agent, 53 mM trehalose, 130 mM sodium chloride, 10 mM methionine and 0.02 (w/v) polysorbate 20, pH 7.0;
(29) 150 units/ml recombinant human HAase, 10 mM phosphate buffering agent, 53 mM trehalose, 130 mM sodium chloride, 10 mM methionine and 0.02 (w/v) polysorbate 20, pH 7.0;
(30) 1000 units/ml recombinant human HAase, 10 mM phosphate buffering agent, 53 mM trehalose, 130 mM sodium chloride, 10 mM methionine and 0.02 (w/v) polysorbate 20, pH 7.0;
(31) 50000 units/ml recombinant human HAase, 10 mM phosphate buffering agent, 53 mM trehalose, 130 mM sodium chloride, 10 mM methionine and 0.02 (w/v) polysorbate 20, pH 7.0;
(32) 300000 units/ml recombinant human HAase, 10 mM phosphate buffering agent, 53 mM trehalose, 130 mM sodium chloride, 10 mM methionine and 0.02 (w/v) polysorbate 20, pH 7.0;
(33) 1500000 units/ml recombinant human HAase, 10 mM phosphate buffering agent, 53 mM trehalose, 130 mM sodium chloride, 10 mM methionine and 0.02 (w/v) polysorbate 20, pH 7.0;
(34) 3000000 units/ml recombinant human HAase, 10 mM phosphate buffering agent, 53 mM trehalose, 130 mM sodium chloride, 10 mM methionine and 0.02 (w/v) polysorbate 20, pH 7.0;
(35) 4500000 units/ml recombinant human HAase, 10 mM phosphate buffering agent, 53 mM trehalose, 130 mM sodium chloride, 10 mM methionine and 0.02 (w/v) polysorbate 20, pH 7.0;
(36) 4500000 units/ml recombinant human HAase, 10 mM phosphate buffering agent, 145 mM sodium chloride, 10 mM methionine and 0.02 (w/v) polysorbate 20, pH 7.0;
(37) 1500000 units/ml recombinant human HAase, 10 mM phosphate buffering agent, 145 mM sodium chloride, 10 mM methionine and 0.02 (w/v) polysorbate 20, pH 7.0;
(38) 300000 units/ml recombinant human HAase, 10 mM phosphate buffering agent, 145 mM sodium chloride, 10 mM methionine and 0.02 (w/v) polysorbate 20, pH 7.0;
(39) 5000 units/ml recombinant human HAase, 10 mM phosphate buffering agent, 145 mM sodium chloride, 10 mM methionine and 0.02 (w/v) polysorbate 20, pH 7.0;
(40) 150 units/ml recombinant human HAase, 10 mM phosphate buffering agent, 145 mM sodium chloride, 10 mM methionine and 0.02 (w/v) polysorbate 20, pH 7.0;
(41) 45 units/ml recombinant human HAase, 10 mM phosphate buffering agent, 145 mM sodium chloride, 10 mM methionine and 0.02 (w/v) polysorbate 20, pH 7.0;
(42) 45 units/ml recombinant human HAase, 10 mM phosphate buffering agent, 22 mM mannitol, 53 mM trehalose, 10 mM methionine and 0.02% polysorbate 20, pH 7.0;
(43) 500 units/ml recombinant human HAase, 10 mM phosphate buffering agent, 22 mM mannitol, 53 mM trehalose, 10 mM methionine and 0.02% polysorbate 20, pH 7.0;
(44) 5000 units/ml recombinant human HAase, 10 mM phosphate buffering agent, 22 mM mannitol, 53 mM trehalose, 10 mM methionine and 0.02% polysorbate 20, pH 7.0;
(45) 50000 units/ml recombinant human HAase, 10 mM phosphate buffering agent, 22 mM mannitol, 53 mM trehalose, 10 mM methionine and 0.02% polysorbate 20, pH 7.0;
(46) 300000 units/ml recombinant human HAase, 10 mM phosphate buffering agent, 22 mM mannitol, 53 mM trehalose, 10 mM methionine and 0.02% polysorbate 20, pH 7.0;
(47) 4500000 units/ml recombinant human HAase, 10 mM phosphate buffering agent, 22 mM mannitol, 53 mM trehalose, 10 mM methionine and 0.02% polysorbate 20, pH 7.0;
(48) 5000 units/ml recombinant human HAase, 5 mM phosphate buffering agent, 53 mM trehalose, 220 mM mannitol, 10 mM methionine and 0.02% polysorbate 20, pH 7.0;
(49) 5000 units/ml recombinant human HAase, 10 mM phosphate buffering agent, 53 mM trehalose, 220 mM mannitol, 10 mM methionine and 0.02% polysorbate 20, pH 7.0;
(50) 5000 units/ml recombinant human HAase, 50 mM phosphate buffering agent, 53 mM trehalose, 220 mM mannitol, 10 mM methionine and 0.02% polysorbate 20, pH 7.0;
(51) 5000 units/ml recombinant human HAase, 5 mM phosphate buffering agent, 53 mM trehalose, 220 mM mannitol, 10 mM methionine and 0.02% polysorbate 20, pH 6.0;
(52) 5000 units/ml recombinant human HAase, 50 mM phosphate buffering agent, 53 mM trehalose, 220 mM mannitol, 10 mM methionine and 0.02% polysorbate 20, pH 6.0;
(53) 5000 units/ml recombinant human HAase, 50 mM acetic acid buffering agent, 53 mM trehalose, 220 mM mannitol, 10 mM methionine and 0.02% polysorbate 20, pH 5.0;
(54) 5000 units/ml recombinant human HAase, 5 mM acetic acid buffering agent, 53 mM trehalose, 220 mM mannitol, 10 mM methionine and 0.02% polysorbate 20, pH 5.0;
(55) 5000 units/ml recombinant human HAase, 50 mM citric acid buffering agent, 53 mM trehalose, 220 mM mannitol, 10 mM methionine and 0.02% polysorbate 20, pH 6.5;
(56) 5000 units/ml recombinant human HAase, 5 mM citric acid buffering agent, 53 mM trehalose, 220 mM mannitol, 10 mM methionine and 0.02% polysorbate 20, pH 6.5;
(57) 5000 units/ml recombinant human HAase, 50 mM Tris buffering agent, 53 mM trehalose, 220 mM mannitol, 10 mM methionine and 0.02% polysorbate 20, pH 8.0;
(58) 5000 units/ml recombinant human HAase, 5 mM Tris buffering agent, 53 mM trehalose, 220 mM mannitol, 10 mM methionine and 0.02% polysorbate 20, pH 8.0;
(59) 5000 units/ml recombinant human HAase, 10 mM phosphate buffering agent, 53 mM trehalose, 220 mM mannitol, 50 mM methionine and 0.02% polysorbate 20, pH 7.0;
(60) 5000 units/ml recombinant human HAase, 10 mM phosphate buffering agent, 53 mM trehalose, 220 mM mannitol, 5 mM methionine and 0.02% polysorbate 20, pH 7.0;
(61) 5000 units/ml recombinant human HAase, 10 mM phosphate buffering agent, 150 mM mannitol, 170 mM sodium chloride, 10 mM methionine and 0.02% polysorbate 20, pH 7.0;
(62) 5000 units/ml recombinant human HAase, 10 mM phosphate buffering agent, 220 mM mannitol, 53 mM trehalose, 10 mM methionine and 0.02% polysorbate 20, pH 7.0;
(63) 5000 units/ml recombinant human HAase, 10 mM phosphate buffering agent, 150 mM mannitol, 50 mM sodium chloride, 53 mM trehalose, 10 mM methionine and 0.02% polysorbate 20, pH 7.0;
(64) 5000 units/ml recombinant human HAase, 10 mM phosphate buffering agent, 150 mM mannitol, 90 mM sodium chloride, 130 mM trehalose, 10 mM methionine and 0.02% polysorbate 20, pH 7.0;
(65) 5000 units/ml recombinant human HAase, 10 mM phosphate buffering agent, 150 mM mannitol, 25 mM trehalose, 10 mM methionine and 0.02% polysorbate 20, pH 7.0;
(66) 5000 units/ml recombinant human HAase, 10 mM phosphate buffering agent, 220 mM mannitol, 53 mM sucrose, 10 mM methionine and 0.02% polysorbate 20, pH 7.0;
(67) 5000 units/ml recombinant human HAase, 10 mM phosphate buffering agent, 150 mM mannitol, 50 mM sodium chloride, 53 mM sucrose, 10 mM methionine and 0.02% polysorbate 20, pH 7.0;
(68) 5000 units/ml recombinant human HAase, 10 mM phosphate buffering agent, 150 mM mannitol, 90 mM sodium chloride, 130 mM sucrose, 10mM methionine and 0.02% polysorbate 20, pH 7.0;
(69) 5000 units/ml recombinant human HAase, 10 mM phosphate buffering agent, 150 mM mannitol, 25 mM sucrose, 10 mM methionine and 0.02% polysorbate 20, pH 7.0;
(70) 5000 units/ml recombinant human HAase, 10 mM phosphate buffering agent, 132 mM trehalose, 150 mM mannitol, 10 mM methionine and 0.02% polysorbate 20, pH 7.0;
(71) 5000 units/ml recombinant human HAase, 10 mM phosphate buffering agent, 26 mM trehalose, 280 mM mannitol, 10 mM methionine and 0.02% polysorbate 20, pH 7.0;
(72) 5000 units/ml recombinant human HAase, 10 mM phosphate buffering agent, 53 mM trehalose, 220 mM mannitol, 10 mM methionine and 0.01% polysorbate 20, pH 7.0;
(73) 5000 units/ml recombinant human HAase, 10 mM phosphate buffering agent, 53 mM trehalose, 220 mM mannitol, 10 mM methionine and 0.10% polysorbate 20, pH 7.0;
(74) 5000 units/ml recombinant human HAase, 10 mM phosphate buffering agent, 53 mM trehalose, 220 mM mannitol, 10 mM methionine and 0.01% polysorbate 80, pH 7.0;
(75) 5000 units/ml recombinant human HAase, 10 mM phosphate buffering agent, 53 mM trehalose, 220 mM mannitol, 10 mM methionine and 0.10% polysorbate 80, pH 7.0;
(76) 5000 units/ml recombinant human HAase, 10 mM phosphate buffering agent, 53 mM trehalose, 220 mM mannitol, 10 mM methionine and 0.02% polysorbate 80, pH 7.0;
(77) 5000 units/ml recombinant human HAase, 10 mM phosphate buffering agent, 53 mM trehalose, 220 mM mannitol, 10 mM methionine and 0.10% Poloxamer 188, pH 7.0;
(78) 5000 units/ml recombinant human HAase, 10 mM phosphate buffering agent, 53 mM trehalose, 220 mM mannitol, 10 mM methionine and 0.01% Poloxamer 188, pH 7.0;
(79) 5000 units/ml recombinant human HAase, 10 mM phosphate buffering agent, 53 mM trehalose, 220 mM mannitol, 10 mM methionine and 0.02% Poloxamer 188, pH 7.0;
(80) 150 units/ml recombinant human HAase, 10 mM phosphate buffering agent, 53 mM trehalose, 220 mM mannitol, 10 mM methionine and 0.02% polysorbate 20, pH 7.0;
(81) 500 units/ml recombinant human HAase, 10 mM phosphate buffering agent, 53 mM trehalose, 220 mM mannitol, 10 mM methionine and 0.02% polysorbate 20, pH 7.0;
(82) 1000 units/ml recombinant human HAase, 10 mM phosphate buffering agent, 53 mM trehalose, 220 mM mannitol, 10 mM methionine and 0.02% polysorbate 20, pH 7.0;
(83) 300000 units/ml recombinant human HAase, 10 mM phosphate buffering agent, 53 mM trehalose, 220 mM mannitol, 10 mM methionine and 0.02% polysorbate 20, pH 7.0;
(84) 50000 units/ml recombinant human HAase, 10 mM phosphate buffering agent, 53 mM trehalose, 220 mM mannitol, 10 mM methionine and 0.02% polysorbate 20, pH 7.0;
(85) 1500000 units/ml recombinant human HAase, 10 mM phosphate buffering agent, 53 mM trehalose, 220 mM mannitol, 10 mM methionine and 0.02% polysorbate 20, pH 7.0;
(86) 3000000 units/ml recombinant human HAase, 10 mM phosphate buffering agent, 53 mM trehalose, 220 mM mannitol, 10 mM methionine and 0.02% polysorbate 20, pH 7.0; and
(87) 4500000 units/ml recombinant human HAase, 10 mM phosphate buffering agent, 53 mM trehalose, 220 mM mannitol, 10 mM methionine and 0.02% polysorbate 20, pH 7.0.

8. The formulation according to any one of claims 1 to 7 for use in the assistance of a drug for intradermal or subcutaneous administration.

9. The use according to claim 8, wherein the drug for intradermal or subcutaneous administration comprises a drug carrier and/or a therapeutic drug.

10. The use according to claim 9, wherein the drug carrier is selected from the group consisting of: a glucose solution, an isotonic electrolyte solution, an alkaline solution, a hypertonic solution, dextran, a syrup substitute and a blood product.

11. The use according to claim 9, wherein the drug carrier is deemed as an agent having therapeutic effect in clinical use in some cases.

12. The use according to claim 9, wherein the therapeutic drug is selected from the group consisting of: chemotherapeutic agents, analgesics, anti-inflammatory agents, antimicrobial agents, anti-amoebic agents, trichomonacidal agents, anti-parkinsonism agent, anti-dysentery agents, antispasmodic agents, antidepressants, antirheumatic agents, antifungal agents, antihypertensive agents, antipyretics, antiparasitics, antihistamines, α-adrenergic agonists, α-blocking agents, anesthetics, bronchodilators, biocidal agents, bactericides, bacteriostatic agents, β-adrenergic blocking agents, calcium channel blocking agents, cardiovascular agents, contraceptives, decongestants, diuretics, sedatives, diagnostic agents, electrolyte agents, hypnotic agents, hormones, hyperglycemic agents, muscle relaxants, muscle contracting agents, ophthalmic agents, parasympathomimetics, psychostimulants, tranquilizers, sympathomimetics, tranquilizer, urinary agents, vaginal agents, antiviral agents, vitamin agents, non-steroidal anti-inflammatory agents, angiotensin converting enzyme inhibitors, polypeptides, proteins, nucleic acids, organic molecules, sleep promoters, insulin, cytokines, antibodies and monoclonal antibodies.

13. A drug composition or a kit, comprising the formulation according to any one of claims 1 to 7 and a drug for intradermal or subcutaneous administration.

14. The drug composition or the kit according to claim 13, wherein the drug for intradermal or subcutaneous administration comprises a drug carrier and/or a therapeutic drug;
preferably, the drug for intradermal or subcutaneous administration comprising comprises a drug carrier and a therapeutic drug, wherein the therapeutic drug is:
(1) separately packaged;
(2) packaged in combination with a drug carrier; or
(3) packaged in combination with a drug carrier and the formulation according to any one of claims 1 to 7.

15. The drug composition and the kit according to claim 13 or 14, wherein the therapeutic drug is selected from the group consisting of: a recombinant protein, a fusion protein, human albumin, immunoglobulin, a targeting antibody drug, a polypeptide, nanoparticles, viruses, cells and chemicals, wherein the polypeptide is preferably selected from: insulin, glucagon-like peptide-1 (GLP-1) analog, leuprorelin, vasopressin, and bacitracin; or
the therapeutic drug is preferably a drug having anti-tumor effect;
preferably, the drug having anti-tumor effect is selected from one or more of oncolytic viruses, immune cells, CAR-T cells, TCR-T cells, anti-tumor drugs, antibody drugs targeting tumors, ADC antibody drugs, immune checkpoint inhibitors, nanoparticle drugs and corticosteroids;
preferably, the antibody drug targeting tumor is selected from one or two of trastuzumab, rituximab, pertuzumab, daratumumab and Isatuximab, for example, rituximab, trastuzumab and pertuzumab, and the concentration of the antibody is preferably 60 to 120 mg/ml;
preferably, when the pertuzumab and trastuzumab are used in combination, the dose of pertuzumab is 300 to 1500 mg, and preferably 600 mg or 1200 mg; and the dose of trastuzumab is 200 to 800 mg, and preferably 600 mg; and
when rituximab is used alone, the dose of rituximab is 700 to 1600 mg, and preferably 1400 mg or 1600 mg.

16. A method for treating a disease, comprising intradermally or subcutaneously administering the formulation according to any one of claims 1 to 7 or the drug composition or the kit according to any one of claims 13 to 15 to a subject.

17. The method according to claim 16, wherein comprises:
(a) intradermally or subcutaneously administering the formulation according to any one of claims 1 to 7 to the subject; and
(b) intradermally or subcutaneously administering the drug for intradermal or subcutaneous administration to the subject,
wherein Steps (a) and (b) perform one or multiple times simultaneously or sequentially in any order; preferably, when Steps (a) and (b) perform sequentially, the time interval between the Steps (a) and (b) is 0 to 24 hrs;
preferably, the time interval is 0 min, at most 1 min, at most 2 min, at most 3 min, at most 4 min, at most 5 min, at most 6 min, at most 7 min, at most 8 min, at most 9 min, at most 10 min, at most 15 min, at most 20 min, at most 25 min, at most 30 min, at most 1 hr, at most 2 hrs, at most 3 hrs, at most 6 hrs, at most 12 hrs or at most 24 hrs.

18. The method according to claim 17, wherein in the case of Steps (a) and (b) perform sequentially, the time interval between Steps (a) and (b) is less than 10 min, and preferably 0 min, 1 min, 2 min, 3 min, 4 min, 5 min, 6 min, 7 min, 8 min or 9 min.

19. The method according to any one of claims 16 to 18, wherein Steps (a) and (b) performing simultaneously or sequentially is enabled by means of a three-way valve; and/or
administration rates the Steps (a) and (b) are controlled by an injection pump or by gravity;
preferably, wherein the administration rate of the Step (a) is 0.1 to 2 ml/min;
preferably, the administration rate of the Step (b) is 5 ml/hr, 10 ml/hr, 30 ml/hr, 60 ml/hr, 120 ml/hr, 240 ml/hr or 300 ml/hr.

20. The method according to claim 16, wherein comprises:
(a) preparing a solution for administration by mixing the formulation according to any one of claims 1 to 7 with a drug for intradermal and subcutaneous administration; and
(b) intradermally or subcutaneously administering the solution for administration to a subject.

21. The method according to claim 16, wherein comprises:
(a) preparing a solution for administration by mixing the formulation according to any one of claims 1 to 7 with a drug carrier and/or a therapeutic drug; and
(b) intradermally or subcutaneously administering the solution for administration to a subject.

22. The method according to claim 16, wherein comprises:
(a) preparing a solution for administration by mixing the formulation of according to any one of claims 1 to 7 with a drug carrier;
(b) intradermally or subcutaneously administering the solution for administration to a subject; and
(c) intradermally or subcutaneously administering a therapeutic drug to a subject.
wherein Steps (b) and (c) perform one or multiple times simultaneously or sequentially in any order; preferably, when Steps (b) and (c) perform sequentially, the time interval between the Step (b) and (c) is 0 to 24 hrs;
preferably, the time interval is 0 min, at most 1 min, at most 2 min, at most 3 min, at most 4 min, at most 5 min, at most 6 min, at most 7 min, at most 8 min, at most 9 min, at most 10 min, at most 15 min, at most 20 min, at most 25 min, at most 30 min, at most 1 hr, at most 2 hrs, at most 3 hrs, at most 6 hrs, at most 12 hrs or at most 24 hrs.

23. The method according to claim 22, wherein in the case of Steps (a) and (b) perform sequentially, the time interval between Steps (a) and (b) is less than 10 min, and preferably 0 min, 1 min, 2 min, 3 min, 4 min, 5 min, 6 min, 7 min, 8 min or 9 min.

24. The method according to claim 22 or 23, wherein Steps (b) and (c) performing simultaneously or sequentially is enabled by means of a three-way valve; and/or
administration rates of the Steps (b) and (c) are controlled by an injection pump or by gravity;
preferably, wherein the administration rate of the Step (b) is 0.1 to 2 ml/min;
preferably, the administration rate of the Step (c) is 5 ml/hr, 10 ml/hr, 30 ml/hr, 60 ml/hr, 120 ml/hr, 240 ml/hr or 300 ml/hr.

25. The method according to any one of claims 16 to 24, wherein the drug for intradermal or subcutaneous administration is preferably in the form of a liquid or a dry powder.

26. The method according to any one of claims 16 to 25, wherein the subject is selected from the group consisting of: a subject having local edema or hematoma after surgery or trauma, a subject receiving local anesthetics, a subject receiving infusion, and a subject receiving the injection of the contrast agent barium sulfate; and
preferably, the subject has accumulated drug solution, exudate, or blood locally in the eyes, vitreous opacities, chemical burn of conjunctiva accompanied by inflammation, traumatic orbital hemorrhage, or traumatic retinal edema.
